# EUROPEAN PATENT APPLICATION

(11) **EP 3 392 343 A1**
(43) Date of publication of application: **24.10.2018**
(21) Application number: 16874165.0
(22) Date of filing: 19.12.2016
(51) Int. Cl.: C12Q 1/06, G01N 33/52, C07D 279/18, C07D 311/82, G01N 1/38

(54) **WATER-BASED CELL STAINING COMPOSITION, METHOD FOR PREPARING SAME AND USE THEREOF, CELL SAMPLE PREPARATION AND CELL COUNTING METHODS**

(30) Priority: 17.12.2015 WO PCT/BR2015/000191
(71) Applicant: Quintino De Oliveira, Henrique Jesus, CEP: 08880-000 Cidade de Mogi das Cruzes - SP (BR)
(72) Inventor: APARECIDA DE OLIVEIRA NASCIMENTO, Vanilda, CEP: 03227-150 São Paulo - SP (BR); FAUSTINO DE MORAES, Tatiane, CEP: 12305-650 Jacarei - SP (BR); QUINTINO DE OLIVEIRA, Henrique Jesus, CEP: 08880-000 Cidade de Mogi das Cruzes (BR)
(74) Representative: Aera A/S
(86) International application number: PCT/BR2016/000163
(87) International publication number: WO 2017/100882

(57) **Abstract**

The present invention relates to a cell dye composition characterized in that it comprises methyl green or an equivalent of its chemical group triarylmethanes (or triphenylmethanes), pironin or an equivalent of its chemical group xanthenes, and fluorescein or an equivalent of its chemical group fluoroma, or salts and complexes thereof. The dye compositions are typically present in a aqueous medium.

## Description

### BACKGROUND OF THE INVENTION

### Blood cells

Blood cells can be classified into three groups: red cells (red blood cells), white blood cells (leukocytes) and platelets (thrombocytes). As blood permeates the entire animal body, these cells are transferred to other parts of it, in particular to body fluids.

Leukocytes represent an important group of cells that are involved in the body defense against infectious agents and foreign substances. Therefore, they are present in various body fluids, in addition to blood, urine, cerebrospinal fluid (CSF), cavity liquids, such as ascites, pleural, peritoneal and synovial; and food fluids, such as milk. Excess leucocytes in animal milk, for example, may represent a major public health problem or financial loss, as it indicates the presence of different infections that can contaminate the entire batch of collected milk due to a diseased animal Pyörälä (2003); Normative Instruction No. 51 of 2011, of the Ministry of Agriculture. Brazilian Sanitary Surveillance Agency Directive.

Human blood in normal state contains 4000 to 10000 leukocytes per cubic millimeter of blood. Under normal health conditions, mammals have five types of leukocytes: neutrophils, basophils, eosinophils, lymphocytes and monocytes (MIRČIČ, 2006; RAWAT, 2015). Leukocytes containing granules are named granulocytes, represented by neutrophils, eosinophils and basophils. Cells without granules are called agranulocytes, represented by lymphocytes and monocytes (MARIEB, 2011). The distribution of white cells typically occurs in the following ranges: neutrophils occur between 40% and 60%; lymphocytes between 20% and 40%; monocytes between 2% and 8%; eosinophils between 1% and 4%; and basophils between 0.1% and 1%, as described by MATHUR et al, 2013. Variations in the total amount of leukocytes and/or ratios of each type are indicative of the presence of a large number of diseases.

The specific concentrations of leukocytes in different body fluids help specialists to discriminate the presence or absence of large groups of pathologies that are important in determining the health status of the subject (PIURI, 2004). When an infection occurs, the production of these cells increases (MARIEB, 2011). According to Ducrest (2005) the leukocyte differential count has a long tradition as a diagnostic tool in human or veterinary medicine, aiding in decision making by the clinician. Counts high or below normal numbers may indicate the presence of many different forms and types of diseases.

Neutrophils are the most abundant leucocytes in the human immune system (CARRERAS, 1994). They are rounded cells, ranging in size from 12 to 14 µm in diameter (RAWAT et al, 2015). They have a lobulated nucleus, which usually contains two to five lobes, connected by a thin chromatin thread. The mature neutrophil is defined by the round lobes showing condensed chromatin, and sometimes the chromatin thread may not be visible, as they are superimposed by the nuclear lobes. Chromatin is coarse, grouped in portions. The cytoplasm is clear and contains several small granules evenly distributed throughout the cell, though they may not be visible when they are over the nucleus. These granules are peroxidase-positive and peroxidase-negative in a ratio of two to one (BEUTLER et al, 2001).

Lymphocytes represent a heterogeneous group of cells that can be easily distinguished from other leukocytes, by their characteristic nuclear and cytoplasmic morphology. However, the general morphology is shared by its three main types: T cells, B cells and natural killer cells (NK) (BEUTLER et al, 2001). Classical blood studies evidence lymphocytes as spherical and ovoid cells with diameters ranging from 5 µm to 17 µm (RAWAT et al, 2015). Lymphocytes are considered small when they have 6 to 9 µm in diameter, whereas large lymphocytes have a diameter of 9 to 17 µm. Small lymphocytes, when stained by Romanowsky derivatives (Wright, Giemsa, or others) have an oval or kidney-shaped nucleus, which blushes in purple, and occupies about 90% of the cell area. The small cytoplasmic border stains in light blue. They have nucleoli, which are rarely observed in the blades of blood distensions when stained with Giemsa (BEUTLER et al, 2001).

Monocytes are the largest normal cells in the blood, whose diameter ranges from 14 µm to 24 µm (RAWAT et al, 2015). The nucleus can be rounded, kidney-shaped, oval or lobed, and may also be folded. The chromatin is organized in thin strands with well-defined margins. When stained with Romanowsky derivatives, the cytoplasm becomes light blue or gray and often vacuolated, especially on slides prepared from blood collected with the anticoagulant EDTA. The gray color of the cytoplasm (as opposed to blue) is due to fine granulation containing RNA (blue staining) and helps to distinguish between monocytes and reactive lymphocytes. The nuclear chromatin of the monocytes contains a fine structure, unlike the coarser clusters of lymphoid chromatin (BEUTLER et al, 2001) .

Eosinophils were first described in 1846 by Jones (McEWEN, 1992), and since then their presence has been associated with parasitic infections, as well as in several other diseases, including dermatoses, allergies, polyarteritis and some neoplastic diseases (BASTEN et al, 1970). DE LA RUE (2001) describes eosinophils as cells slightly larger than neutrophils. Their diameter is between 12 µm and 15 µm (RAWAT et al, 2015). Usually the nucleus has only two lobes. The chromatin pattern is the same found in neutrophils, but the nucleus tends to be more slightly stained. The main distinguishing feature of these cells is the presence of many refringent granules, which the Romanowsky derivatives stain in reddish orange and are evenly distributed throughout the cell (Beutler et al., 2001). The granules contain mainly the major alkaline protein (MAP), eosinophilic cationic protein (ECP) and eosinophil peroxidase (EOP) (SPRY, 1985; McEWEN, 1992; SPRY, 1992; STEVENS, LOWE 1993; TIZARD, 1998) .

Basophils represent a population of granulocytic cells morphologically distinct in blood and are the main mediators of allergic inflammation (KURIMOTO, 1989; FALCONE, 2000). Basophils are less numerous, accounting for less than 1% of the differential count. Their size is intermediate between neutrophilic and eosinophilic granulocytes, ranging from 10 µm to 14 µm in diameter (RAWAT et al, 2015). With Wright stain (which is also a Romanowsky derivative), they are easily recognizable because of their large and abundant granules. These granules are basophilic and becomes dark blue colored (OVALLE, NAHIRNEY, 2014). The nucleus, generally irregular or bilobed, is less evident, since it is hidden by the granules (ANDREO FILHO, 2009).

It is important to classify and count blood cells and other body fluids (especially leukocytes, which are indicative of many infectious processes) for assessing the health status of the individual under numerous conditions.

As examples of criteria used to classify leukocytes in imaging tests, one can cite differences in texture, affinity with different dyes, size, content and morphologies of the nucleus and cytoplasm of each cell type.

The counting and characterization of blood cells, or hemogram, is the most requested laboratory testing for monitoring and screening the patient's health status. Blood count and morphological evaluation of blood cells have been an important tool not only for the diagnosis of diseases, but also as an "proof" of the patient's overall health status in periodic testings and in check-ups (ROSENFELD, 2012). Particularly, the leukocyte differential count has a long tradition as a diagnostic tool in human or veterinary medicine, aiding in decision making by the clinician (DUCREST, 2005). Therefore, over the years, information derived from differential leukocyte counts have become paramount in the diagnostic evaluation of the patient, and are also routinely used for screening and monitoring hematological and non-hematological diseases.

### Hemogram and methods of staining and counting cells

The identification and differentiation of leukocytes in various human or animal body fluids such as blood, urine, milk, cerebrospinal fluid and cavity liquids, such as ascites, pleural, peritoneal and synovial, require different types of tests. All these fluids are predominantly aqueous; however, all stains that are used to differentiate leukocytes are available only in alcoholic solutions. Since the differential dyes are in an alcoholic medium, the body fluid to be stained necessarily has to be distended on a slide, dried, fixed, and then be subjected to differential staining of the leukocytes.

When these dye solutions are inserted directly into the aqueous fluids, the staining may even occur, as with Leishiman, Wrigth and Giemsa solutions; however, particle agglutination occurs, which makes proper count or classification of the cells impossible.

There are dyes which make the differential staining of leukocytes in aqueous medium, but they are used in flow cytometers for differentiation only in blood (Brazilian Sysmex Patent No. 11 2013 027349. Such dyes are not used to differentiate cells under a microscope, as they do on distension slides, and they do not serve to differentiate leukocytes in other fluids other than blood. Therefore, they have never been used in accordance with the proposal of this invention.

### STATE OF THE ART

### Dyes and methods for coloring cells

Currently, all cell dye compositions that allow differential reading of leukocytes under a microscope, in a reliable manner, are produced in alcoholic medium. The only reliable results of these dyes are obtained when they are applied in fluid distensions, on a glass slide, and fixed with some alcoholic permeabilizer, such as methanol.

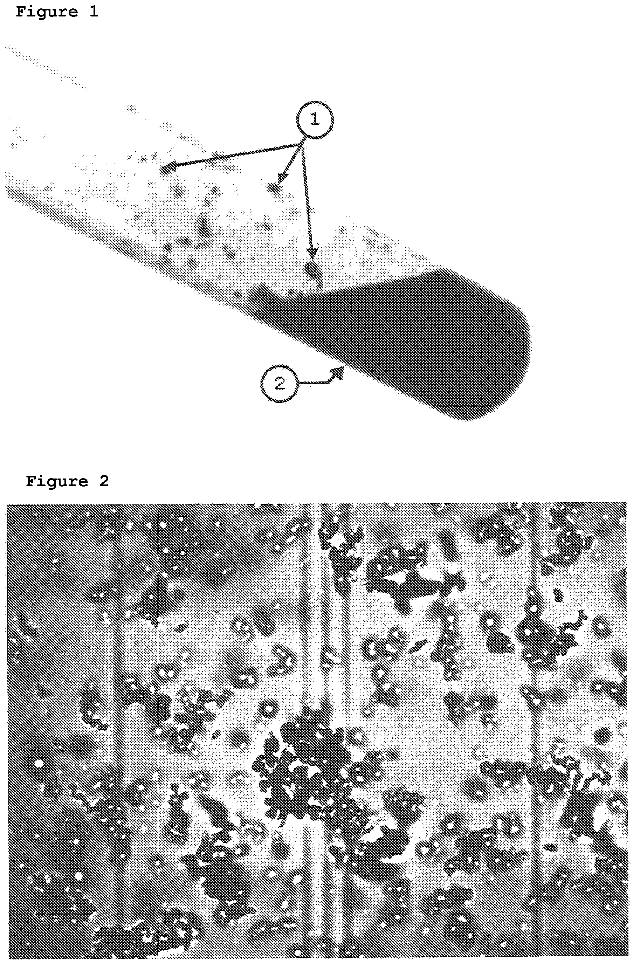

Some of the presently available dye compositions may perform differential staining in liquid alcohol medium. However, when body fluid is placed in an alcoholic liquid medium, coagulation or agglutination of solids forming lumps occurs as it is shown in the integral solution in figure 1, or as it is shown in detail in the image obtained in a Neubauer chamber shown in figure 2. The results of these colorings are not practical, since the agglutination of the particles makes the counting and correct classification of cells impossible. Thus, it is not possible to separate the cells contained in the agglutinate to perform the counting.

Therefore, there is currently a need in the art for dye compositions which allow the differential staining of cells in non-alcoholic liquid medium or with a lower alcohol content to avoid the above-mentioned problems.

One form of leukocyte staining in non-alcoholic liquid medium is made primarily with methylene blue or its derivatives, for example, forming the "Turk's solution". However, this type of staining does not allow the differentiation of leukocytes into their types and subtypes. This class of dyes allows only determining the presence of leukocytes in the sample, but does not allow differentiating the same in order to carry out a qualitative analysis. Accordingly, an aqueous dye composition, or of a lower alcohol content, capable of performing the differential coloration in a liquid medium has not been described in the prior art.

The most commonly used technique nowadays for laboratory tests requires the preparation of blood distension, which requires skilled and experienced professionals, in addition to taking considerable time to complete. Therefore, in cases of emergency or urgency care, doctors are led to take decisions without the testing of blood count or leukogram, of any of the body fluids. Additionally, in routine consultations, when a test request is made, the patient should perform it in a clinical laboratory at a later time and return with the test result for evaluation by the physician. This approach demands too much time for all involved.

In order to differentiate leukocytes, for example, by the manual counting method, it is necessary to prepare a whole blood, or any other body fluid of interest, distension on a new slide. Some large laboratories have automatic equipment to carry out and prepare blood distention. However, these are not broadly available. In addition, such automatic equipment do not distend other body fluids. Therefore, typically, the distension of any body fluid is prepared by a laboratory biologist, biochemist, physician or biomedical professional. However, in order to achieve distension with acceptable quality, this professional must have specific training and good experience with the technique. After distension, the sample should be dried naturally and then fixed with some type of alcohol (for example, ethanol or methanol). Once fixed, the distension should be stained by specific dyes.

The dyes used for blood distension were developed by the Russian doctor Dmitri L. Romanowsky (1861-1921) at the end of the 19th century. After that, several derivatives of the same dye emerged, which act according to the same principles, obtaining similar results. Such are the dyes known as: Wright, Giemsa, Leshiman, May-Grunwald and Rosenfeld, among others. These dyes are so old that there is no patent pending or in effect for them. Besides of being derived from Romanowsky, they all have another characteristic in common: they act only on fixed distention. None of these, or any other dye known to date, is capable of staining the leukocytes in liquid medium so as to allow differentiation and counting thereof, by expert visual evaluation or by image processing. This is the reason why such dyes: (i) do not stain the cells differentially, and (ii) those that stain differently do not allow counting because of the clumps of particles that have been removed and are in suspension, leading to the loss of amount reference of cells per volume of fluid.

Dyes based on the Romanowsky formulation and the manual method used to differentiate leukocytes are widely used in hematology. However, the staining for visual differentiation has not been methodologically improved for several decades. Therefore, there is a need in the art for dye compositions which differentially stain cells and do not form cell clumps, allowing counting the same.

Another important fact is that none of these dyes or method used for manual differential counting eliminates the presence of red blood cells, which are also stained. The presence of red blood cells impairs the work of specialists, who need to get leukocytes dispersed in the midst of a myriad of red blood cells (the normal proportion is 1,000 red cells for each leukocyte). The presence of red blood cells has little effect on the work of experienced specialists who have learned to ignore them. However, it dramatically affects leukocyte targeting and recognition algorithms in image processing programs. For this reason, there is a great need in the art for a dye that eliminates or does not color the red blood cells and, at the same time, preserves the leukocytes stained differently.

Romanowsky-derived stains have as their basic principle that acidic cell structures are stained by alkaline dyes and that alkaline structures are stained by acid dyes. The alkaline dyes are represented by methylene blue and its derivatives, whereas eosin is usually the acid element of the dye solution (BAIN, 2007; OLIVEIRA, 2007). There is no consensus as to which of these combinations is ideal for use in staining and identification of blood cells (WITTEKIND, 1979) .

Such dyes contain, among their essential components, a mixture in which an alkaline cationic component, such as azur-B, gives the blue or purplish-blue color to nucleic acids, DNA and RNA. This is possible as they bind to the phosphate groups found in acids, nucleoproteins, basophil granules and, to a lesser extent, the eosinophil granules. An anionic acid dye, also present in the mixture, imparts an orange color to the hemoglobin, to the eosinophilic granules and also helps in the nucleus staining, since it is also combined with the nuclei proteins. Eosin is a dye that meets this requirement and is usually combined with azur-B (WITTEKIND, 1979) or methylene blue, producing one of the satisfactory dyes derived from Romanowsky (MARSHALL et al, 1975). The stainings produced with these blends are pH-dependent, including that of the slide wash water. They also require filtration before use, otherwise they form particulate deposits on distensions, which can lead to reading errors, such as the visualization of corpuscles in erythrocytes. These dye combinations use methanol as a solvent and diluent, rendering them unsuitable for staining blood cells in liquid media, as they coagulate the blood and do not allow the stained cells to be visualized.

The two main colorants of the solutions that make up the derivatives of Romanowisk are methylene blue and eosin Y, whose characterizations are presented. According to Poggere et al. (2011), methylene blue, whose chemical structure is shown below, is an alkaline dye that belongs to the class of phenothiazines. It is organic, aromatic, heterocyclic, soluble in water or alcohol (LIMA et al., 2007). Alkaline dyes are water soluble and produce colored cations in solution. Thus, they are generally referred to as cationic dyes (POGGERE et al, 2011). It presents maximum absorption at 661 nm in aqueous solution and at 656 nm when in methanol solution (HOROBIN, 2008).

### Chemical structure of the methylene blue dye:

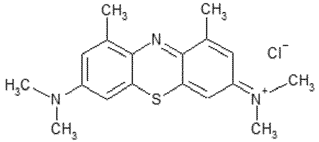

Eosin Y is an anionic hydroxyxanthene dye that exhibits maximum absorption at 516 nm in alkaline or neutral aqueous solution (HOROBIN, 2008). It absorbs at 524 nm in alcohol solution. Its chemical structure is shown in the structure below. It is widely used to stain the cytoplasm and cytoplasmic structures such as eosinophilic granules.

### Chemical structure of eosin Y dye

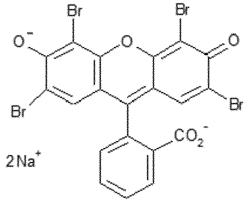

Another class of known dyes, which are used mainly for staining histological sections, but which also stain blood cells, are those with selectivity stain deoxyribonucleic (DNA) and ribonucleic (RNA) acids, such as methyl green (MG) and pironin (P).

MG has a strong cationic character, which causes it to be electrostatically attracted by the phosphate groups, which are located in the outer part of the DNA chain. These results in leukocyte nuclei stained in blue-green.

Pironin is slightly cationic, which gives it greater affinity with flat and less polymerized structures, having high affinity with ribonucleic acid molecules. Thus, it stains the nucleoli and the areas containing RNA in pink tones (PERRY et al, 1956; KIERNAN, 1999; HOROBIN, 2008).

Another dye that can integrate this group is the fluorescein that blends cytoplasmic granules and other structures that contain alkaline proteins, due to its anionic character. Thus, eosinophilic granules assume a yellow-orange coloration. This dye grouping produces three groups of colors, the combination of which allows differentiation of leukocytes into blood distensions.

The three dyes described above present desirable characteristics for leukocyte differentiation in manual tests, as it provides another recognition pathway, which goes beyond morphological analysis, which is the main distinguishing feature in Romanowsky-derivative stains. The only descriptions of such dyes in blood distension were presented by Ornsteien et al. (1974) and TYCKO et al., 1976. According to Tycho et al. this staining has the potential to differentiate leukocytes by imaging. However, it failed as the time of action on blood distension is long (greater than 30 minutes) and the resulting staining is less contrasting than those obtained with the Romanowsky derivatives for visual analysis. These works do not present details of how these dyes are prepared and how they work.

A study published in 2014 by Nascimento, Moraes and Oliveira (2014) resumes this combination of dyes, now in liquid medium and at a concentration about 10 times higher than that of commercial dyes, in order to propose a method of image processing for leukocytes differentiation. The staining solved the problem of the time of action of the dyes, which happened to occur in less than a minute, due to the high concentration. However, although it has potential for use in image processing, the dye does not solve the problem of the particle agglutinates formation, as documented by Tycho et al. (1976), since they are alcoholic, as described in the preparation method. In addition, according to Nascimento, Moraes and Oliveira (2014), in the description of the methods, the validation of the dye, the images acquisition and the cells characterization are done through histograms, and were performed on blood distension slides. It was verified in liquid medium that the staining occurs, but characterizing the cells by histograms was not possible.

In addition, the dye proposed by Nascimento, Moraes and Oliveira (2014) does not have adequate osmotic equilibrium, which results in cells lysing after a few minutes in the solution. Therefore, both the dye and the method were considered inadequate for counting and classification of leukocytes in diagnostic tests. Said scientific article also does not mention the possibility of staining in the absence of the hemolysing agent (H) of the composition.

In view of the above, there is a need in the art for novel dye preparation compositions and methods capable of radically reducing the presence of alcohol in the formulation, rendering the solution predominantly aqueous in order to avoid the formation of particulate clumps; ensure osmotic balance, to prevent cell disruption; allow staining of cells in the absence of the hemolysing agent; and counting and classifying leukocytes directly in diagnostic tests, without the use of cell distension techniques.

Although the dye compositions of the present invention may comprise concentrations and ratios similar to known compositions, it differs from all the foregoing because:
- they present a new formulation, with cyanide-free haemolysant, as recommended by the legislation of several countries;
- they are prepared by a method which stabilizes the composition in aqueous medium and, therefore, modifies all the results obtained in cell staining in liquid medium;
- they do not produce clumps of particles (lumps), but, on the contrary, they produce a clear solution, which allows the exclusive visualization of leukocytes, stained differently; and
- the leukocyte images obtained with the present invention are free from lumps, from other cells such as erythrocytes and from cell debris, which makes differential staining via predominantly aqueous liquid medium feasible.

Additionally, it was possible to establish the osmotic balance so that the cells remain viable for classification and counting for more than 30 minutes. The results obtained demonstrate that the dye compositions and methods of the present invention are suitable for diagnostics. This new composition eliminates the preparation of blood distention and allows simultaneous global and differential counting of leukocytes in appropriate equipment, such as the Neubauer chamber.

As shown above, the dye solutions are currently prepared in alcoholic media, since the alcohol permeabilizes the cell membrane, which facilitates the entry of the dye into the cell. However, there are no records of water-based coloring solutions that render the differential coloration to date, as the problem of cell permeabilization of aqueous-based dye compositions had not been solved. Thus, there was also a need in the art for an aqueous based dye composition with cell permeabilization capability, for the penetration of dyes into the cells. For this reason, the present invention relates to compositions which effect permeabilization of the membrane for penetration of the dyes even in predominantly aqueous medium. Thus, the dyes penetrate the cells, react with the cell structures with which they have affinity, as previously described, and allow the differentiation of leukocytes in an adequate manner.

### Cell counting methods

The counting methods have evolved in the last decades, with the technological sophistication of laboratories. Tests such as erythrogram, leukogram and plaque were replaced by an automated complete blood count, which shows the differential count of leukocytes in the five types, among many other information. The methods incorporated into the automated counting equipment perform differential counting in less than one minute (ROSENFELD, 2012). On the other hand, the manual global and differential counting of leukocytes has remained stagnant and has not evolved for more than 30 years. Attempts to perform automated differential counting using the classic slide method or Neubauer chamber were performed without practical success. (Tycho et al. (1976), Adollah et al. (2008), Mahajan et al. (2014), Oliveira (2007), Oliveira et al. (2014), point out methods that allow the leukocytes to be stained in distension or cause inappropriate staining in the liquid medium. The distension readings are very labor-intensive and costly for clinical laboratories and, so far, there is no alternative to this method, since automated readings do not cover all possible test results.

Therefore, there are currently only two methods available to perform global and differential counting of leukocytes in body fluids: the manual, which is performed from the distension of the fluid sample and the automated one, using flow cytometry. Both require at least two steps: the collection, which is carried out by a professional with the patient and the preparation and reading of blood, which are done by another professional in the laboratory of clinical analysis. Even in a health center that is equipped with ample financial resources and modern automatic equipment, the time between collection and obtaining of the results rarely occurs with less than 60 minutes; whereas in the typical situation, when the patient receives the medical request and is directed to the laboratory, the result is obtained within 48 hours to 72 hours after blood collection.

In addition, when the testing is performed in automated equipment and the results point to deviations from the normal values, it is necessary preparing a new blood sample for manual reading. The automated equipment does not allow verification of the results with the blood sample that was used, since it is discarded after the counts, that is, they neither preserve the cells nor their images for future evaluation. This requires the repetition of the reading by manual technique, by a specialist. And when this occurs, it is worth remembering that manual readings are labor-intensive, time-consuming and require trained and experienced staff, which ultimately increases costs and results takes considerably longer (BAIN, 2007).

In the current state of the art, manual counting in absolute values is done in the Neubauer chamber, staining the leukocytes undifferentiated in liquid medium with Turk's solution or derivatives. The percentage differential count is made in blood distension, the only reliable and accepted method by health professionals and regulatory agencies. Attempts to differential staining in liquid media result in particle agglutination (figure 1) which, in spite of staining, prevents the correct cell counting, since there are no means to determine the cells that are in the liquid agglutinates. In addition, these stains require a long time of exposure to the dye to take effect.

### APPROACH OF THE SOLUTION TO THE PROBLEM

In view of the foregoing, it is evident that obtaining the most rapid blood count during routine visits or in emergency or urgency care is a long-standing technological barrier - and, until the development of the present invention - without a prospect of significant improvement, although it is essential to assess the patient's health status (GROTTO, 2009) .

As previously described, the manual coloring and differential counting techniques of leukocytes have not been improved for decades, although they are widely used. No leukocyte staining method, which is used in manual techniques, performs differential staining in a liquid medium in order to obtain diagnostic results. All types and combinations of dyes only achieve viable stains on slide fixed distensions. Additionally, both methods and dyes used to date keep the erythrocytes intact on the slides, which makes it difficult to count leukocytes, especially in imaging methods.

It is desirable to develop a third method that combines the agility and the low cost of automated reading with the possibility of evaluating the results by the healthcare professional, visualizing the images of the cells, when necessary.

In accordance with such technical need, the present invention provides, in one embodiment, a dye composition capable of differentially staining cells, particularly leukocytes, in an aqueous-based liquid medium, according to the respective types, without forming particle agglutinates and with the possibility of simultaneously performing red cell lysis, if necessary. This novel form of staining provides that this invention also contemplates a novel method of counting the leukocytes in liquid medium, which exempts the preparation of the blood distension, performing the procedure of global and differential counting of the cells simultaneously in Neubauer's chamber or in a similar device.

Suppressing blood distension and performing total and differential counting of leucocytes in liquid media, as done in automated cytometers, and at the same time providing the same characteristics of the images obtained in a stained distension are expressive advances for a technique that has not been improved for decades, but it is fundamental to establish reliable diagnosis. Thus, the aim of this invention is carrying out differential and total counting of leukocytes in one step, by making blood preparation and the differential staining of the cells in liquid medium so that they are counted directly under the microscope and photographed for verification or evaluation of the results, without the need for reading repetitions. For this, a dye composition that penetrates and stains leukocytes in a distinguish manner was developed.

The composition disclosed herein comprises combinations of methyl green (MG), pironin (P) and fluorescein (F) dyes, which may or may not be associated with a lysing agent, forming a dye complex/lysing agent which acts in one step in a liquid medium.

The dye composition of the present invention differentiates leukocytes without the need for morphological analysis of the cells, which represents a major advance for recognition by image processing, and therefore, the cells can be classified into 1000x smaller magnifications, dispensing the use of immersion oil, which further simplifies the counting process.

The method uses simple information obtained from histograms and a logic classification, which is also simple, making it possible to obtain an automated classification that is equivalent to a flow cytometer.

### Dyeing performance

Methyl green acts as a triarylmethane cationic dye that exhibits maximum absorption at 635 nm and a subsidiary peak at 410 nm when diluted in water (HOROBIN, 2008). One of its chemical structures is presented below.

### Chemical structure of MG dye

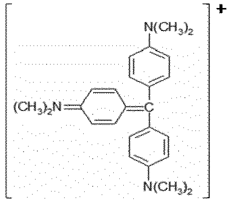

Pironin acts as a mildly cationic dye that has good affinity for RNA. It has maximum absorption peak at 546 nm when diluted in water, whereas in 50% solution of aqueous ethanol, the maximum absorption occurs at 549 nm (HOROBIN, 2008) .

### Chemical structure of pironin dye

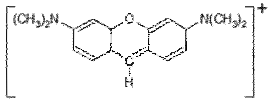

Fluorescein is an anionic dye, which has affinity for cytoplasmic proteins, such as eosinophilic granules. It exhibits maximum absorption at 490 nm when diluted in water. Since pH is important to set forth the range of absorption where it acts, so it is possible to find the absorption peak at other wavelengths if the pH is different. (HOROBIN, 2008; BANCROFT, 2013). Its chemical structure is shown below.

### Chemical structure of fluorescein dye

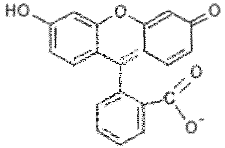

The charge selective dyes (CSDs) reported in the literature deal only with the combination of MG and pironin (ROWLEY *et al*., 2008; PATERNITI *et al*., 2011). Fluorescein is used separately and no research has been found associating it with dyes depending on the charge.

### Comparing the classes of dyes

By comparing the performance of the two classes of dyes, it is possible to establish differences according to the following characteristics: 1) The color spectrum resulting from the cells staining. The Romanowsky derivatives result in the rosy-blue colors for almost all cells (except the eosinophil), implying that the differentiation between them is mainly due to the morphological analysis. On the other hand, CSDs result in three groups of colors, the combination of which allows to select the type of cell (TYCHO et al., 1976). 2) solubility in polar liquids. Romanowsky derivatives are solubilized in non-polar solvents, typically in alcoholic solutions. While CSDs are soluble in both polar and nonpolar solvents. This feature allows blood staining in a liquid medium without coagulation or particle agglutination.

The demonstration of DNA can be performed by different techniques such as those evidencing deoxyribose or by staining with MG and P, in which the phosphate groups combine with the base of the dye MG at acidic pH or can be exhibited by fluorescent methods, using dyes such as acridine orange. On the other hand, the RNA demonstration can be performed by means of acridine orange staining, or other forms requiring complex extraction processes. Thus, the method of choice for evidencing both DNA and RNA is based on the staining technique with MG and P, as described by Bancroft (2013).

The principle of the technique is that both the dyes used in combination are cationic and the MG binds preferentially and specifically to the DNA, allowing P to bind to the RNA. The specific reactivity of MG is attributed to the spatial alignment of its NH₂ groups with the phosphate radicals in the DNA double helix. Coloration by P, on the other hand, does not show this spatial affinity, so that other negatively charged becomes red (pink) stained. Furthermore, as these dyes are soluble in water or aqueous acetate solution, and they also do not coagulate the blood when used in liquid medium, they have useful characteristics when it is desired to perform staining in this medium.

### OBJECTIVES OF THE INVENTION

In a first embodiment, the present invention aims to provide aqueous compositions comprising cell dyes or salts and complexes thereof. Such compositions may further comprise one or more cell lysing agent(s) and an alcohol concentration of less than or equal to 35% by volume.

In a second embodiment, the present invention relates to the process of preparing the composition comprising mixing dyes and, optionally, one or more lysing agent (s) . Preferably, such lysing agents may be cyanide-free.

In a third embodiment, the present invention aims to provide a method of counting cells derived from body fluids stained with the above-described cell dye composition.

In a fourth embodiment, the present invention provides a method of cell counting comprising (i) obtaining an image of the cells suspended in the dye composition; (ii) global cell type counting and differential counting of cell subtypes in a single step, wherein such counts can be made by traditional methods, using a microscope, or by processing the image obtained in step (i).

In a fifth embodiment, the present invention relates to the use of dye compositions for the preparation of a cell sample for cell analysis.

Therefore, the compositions, methods and uses of the present invention are an improvement to the current technique, the manual method, by collecting microsamples of body fluids which are diluted in solution of dyes and lysing agents (where appropriate), enabling the preparation and delivery of the cells for one-step reading. The global and differential counts can be done automatically, by image processing, or by the healthcare professional directly, under a microscope. The digital images of the cells can be stored for possible future evaluations, without the need for new collection and testing.

### DETAILED DESCRIPTION OF THE DISCLOSURE

As described above, the present invention relates, in a first aspect, to cell dye compositions, comprising a mixture of dyes in predominantly aqueous medium. Predominantly aqueous medium means a liquid medium, as a solution, comprising less than 50% alcohol. However, in preferred embodiments of this invention, a predominantly aqueous medium is to be understood as a medium comprising less than 40%, less than 35% or less than 30%, less than 20% or less than 10% alcohol.

Such a mixture has as its main advantages the ability to permeabilize cell membranes for dye penetration and the possibility of preparation of clear cell samples without the presence of artifacts, fragments or lysed erythrocyte lumps that could impair the cell count of interest. In addition, the compositions of the present invention differentially color cell types and subtypes.

Optionally, the compositions of the present invention comprise methyl green, pironin and fluorescein dyes, or salts and complexes thereof, in predominantly aqueous medium. One skilled in the art knows that there are several subtypes of dyes and the three types mentioned above can be replaced by dyes having equivalent physicochemical properties, for example, the dyes of the chemical group of triarylmethanes (or triphenylmethanes), which comprises methyl green, and the dyes of the xanthenes chemical group, where the different forms of pironin are included, such as pironins G, Y, B that form part of the subgroup fluorene and the different forms of fluorescein, such as fluorescein acid, fluorescein sodium and fluorescein diacetate, which are part of the fluorone subgroup.

The compositions of the present invention may optionally comprise a cell lysing agent which, according to the laws and safety standards in force in several countries, should be free of cyanide. A cell lysing agent suitable for the present invention may be selected from quaternary agents, such as quaternary ammonium, which are capable of disrupting the membranes of the red blood cells.

Therefore, the compositions of the present invention comprise from 0.3 mM to 8 mM methyl green or equivalent dye, preferably 4.0 mM to 7.0 mM, 0.5 mM to 7 mM, 5 mM to 8 mM, 0 mM or 1.0 mM to 6 mM, or, for example, 2.0 mM to 5 mM, and optionally from 3.0 mM to 4 mM.

Prionin may be present in a concentration of 0.3 mM to 8 mM, preferably 0.5 mM to 7 mM or 1.0 mM to 6 mM or, for example, from 2.0 mM to 5 mM, and optionally from 3.0 mM to 4 mM.

The fluorescein concentration may vary within the range from 0.1 mM to 3 mM, optionally from 0.3 mM to 2.5 mM or, for example, from 0.5 mM to 2 mM or 1.0 mM to 1.5 mM.

Finally, the concentration of the cell lysing agent, such as quaternary ammonium, if present, in the composition, may range from 25 g/L to 80 g/L diluted in aqueous medium.

The compositions of the present invention have the particularity that the dyes, for example, methyl green, pyronin and fluorescein and, optionally, a lysing agent, form a complex in predominantly aqueous medium or in alcoholic concentration less than or equal to 35%.

In a second embodiment, the present invention discloses the method of obtaining the dye compositions. This method involves three basic steps, namely:
- Preparing a solution of methyl green and pironin, obtaining a MGP solution;
- Adding fluorescein to the MGP solution, obtaining a solution named MGPF; and
- Optionally, adding a lysing agent, obtaining a MGPF(H) solution.

More specifically, methyl green and pironin are mixed in concentrations between 0.3 mM and 8 mM each, preferably 0.5 mM to 7 mM, or 1.0 mM to 6 mM, or, for example, 2.0 mM to 5 mM, optionally 3.0 mM to 4 mM, the MGP solution having a pH of 3.8 to 5.5.

Preferably, Fluorescein is added in a ratio of one part of MGP to two parts of fluorescein in the concentration of 0.1 mM to 3 mM, optionally 0.3 mM to 2.5 mM or, for example, 0.5 mM to 2 mM or 1.0mM to 1.5mM, and two and a half parts of MGP to one part of Fluorescein, thereby obtaining the MGPF solution having a pH between 4.8 and 6.4.

Optionally, the lysing agent may be added to the composition of the present invention in a ratio of a part of lysing agent (in the ratio of 25 g/L to 80 g/L associated in the ratio of 2 g/L to 9 g/L, both previously diluted in aqueous medium to two parts of the MGPF solution to two parts of the lysis to one part of the MGPF solution, wherein the final coloring solution of MGPF(H) should remain at pH between 5.5 and 6.6.

As it has been briefly described above, the present invention further relates to a method of preparing cell samples comprising cell lysis and single-step differential staining of cell types in aqueous medium. Such method comprises the step of mixing a sample of cells with a dye composition or dye/lysing agent, as described above. Such step allows permeabilization of the cell membranes in predominantly aqueous medium.

Preferably, a sample of cells is mixed with a dye composition or dye/lysing agent in a ratio of one part of body fluid to 5 to 200 parts of the dye composition, depending on the type of body fluid to be analyzed. Optionally, a body fluid part to 10 to 150 parts of the dye composition, or a body fluid part to 20 to 130 parts of the dye composition, a body fluid part to 40 to 100 parts of the dye composition or, for example, a part of body fluid to 60 to 90 parts of the dye composition.

In a fourth embodiment, the present invention relates to a method of counting cells comprising the steps of mixing a sample of cells with a dye composition or dye/lysing agent, placing the sample mixture of cells with the dye composition or dye/lysing agent in a cell counting apparatus and, finally, counting the total value of the cell units, identifying and determining the ratios of each cell subtype in the body fluid sample in a single step.

Particularly, the cell counting method of the present invention is characterized in that it comprises (i) imaging the cells suspended in the dye composition; (ii) global cell type counting and differential counting of cell subtypes in a single step.

More particularly, the counting of the cell counting method of the present invention is carried out by image processing, or by a subject using a microscope, thus, such a method may further comprise the step of recording images of the stained and lysed cells and storing such images for future sample evaluations.

In preferred embodiments of the invention, the cell samples used in any of the methods of the invention consist of or are obtained from animal or human body fluids and may comprise red blood cells, leukocytes and thrombocytes, wherein there are selected cell subtypes of neutrophils, basophils, eosinophils, lymphocytes (B and T types) and monocytes, as well as the immature forms of these cells.

In a further embodiment, the present invention relates to the use of a composition, as defined above, for the preparation of a cell sample for cell analysis.

According to the present invention, the sample subjected to the methods of analysis, or for which the dye composition of the present invention is intended, may be a sample obtained from any body fluid, secretions, blood and other tissues. The cells contained in the sample, by their turn, can be cells of any animal.

According to these concepts, various compositions obtained by different processes have been developed and result in satisfactory methods of sample preparation and cell counting. Some examples of compositions and methods falling within the scope of the present invention are provided in this specification, without, however, being intended to limit the scope of protection, since the present invention and its concepts allow the design of compositions comprising different combinations of dyes and cell lysates.

### DESCRIPTION OF THE FIGURES

Figure 1 - Agglutination of blood particles stained by dyes prepared in alcoholic medium, indicating (1) the lumps of blood stained in alcohol solution and (2) whole blood with EDTA in Guiemsa standard solution.
Figure 2 - Formation of lumps of blood diluted and stained with Leishman in alcoholic medium and visualized in Neubauer chamber.
Figure 3 - Absorbance spectra of the individual components of the proposed dye solution and the final solution.
Figure 4 - Absorbance spectra of the final solution of MGPF(H) read on September 19th, 2014, six months after and fourteen months after that is when pironin degradation begins.
Figure 5 - Eosinophils in blood distension stained with MGPF. (a) with magnification of 400x; (b) with magnification of 1,000x.
Figure 6 - Basophil in blood distension stained with MGPF, at magnification of 1000x.
Figure 7 - Neutrophil samples, stained in liquid medium, stained in liquid medium with MGPF(H) and MGPF, obtained from whole blood and synovial fluids. Images obtained with magnification of 1000x.
Figure 8 - Lymphocyte samples, stained in liquid medium with MGPF(H) and MGPF, obtained from whole blood and synovial fluids. Images obtained with magnification of 1000x.
Figure 9 - Monocyte samples, stained in liquid medium, stained in liquid medium with MGPF(H) and MGPF, obtained from whole blood and synovial fluids. Images obtained with magnification of 1000x.
Figure 10 - Eosinophil samples, stained in liquid medium, stained in liquid medium with MGPF(H) and MGPF, obtained from whole blood and synovial fluids. Images obtained with magnification of 1000x.
Figure 11 - Basophil sample, stained in liquid medium with MGPF(H), obtained from whole blood. Images obtained with magnification of 1000x.
Figure 12 - Bland-Altman analysis comparing the global counts performed by automatic equipment in the laboratory of clinical analysis (LCA) and manual with MGPF(H).
Figure 13 - Bland-Altman analysis comparing global counts in a Neubauer chamber with Turk's solution and MGPF(H)
Figure 14 - Bland-Altman analysis comparing the neutrophil differentiating counts performed with an automatic equipment in the LCA and manual with MGPF(H).
Figure 15 - Bland-Altman analysis comparing the differential counts of neutrophils in liquid medium stained with MGPF(H) and in blood distension stained with Leishman.
Figure 16 - Bland-Altman analysis comparing the lymphocyte differentiating counts performed with an automatic equipment in the LCA and manual with MGPF(H).
Figure 17 - Bland-Altman analysis comparing the differential counts of lymphocytes in liquid medium stained with MGPF(H) and in blood distension stained with Leishman.
Figure 18 - Bland-Altman analysis comparing the monocyte differentiating counts performed with an automatic equipment in the LCA and manual with MGPF(H).
Figure 19 - Bland-Altman analysis comparing the differential counts of monocytes in liquid medium stained with MGPF(H) and in blood distension stained with Leishman.
Figure 20 - Bland-Altman analysis comparing the eosinophil differentiating counts performed with an automatic equipment in the LCA and manual with MGPF(H).
Figure 21 - Bland-Altman analysis comparing the differential counts of eosinophils in liquid medium stained with MGPF(H) and in blood distension stained with Leishman.
Figure 22 - RGB histograms obtained from neutrophil images. Wherein (A) are the histograms of R channel; (B) are the histograms of (G) channel; and (C) are the histograms of channel B.
Figure 23 - RGB histograms obtained from lymphocyte images. Wherein (A) are the histograms of R channel; (B) are the histograms of (G) channel; and (C) are the histograms of channel B.
Figure 24 - RGB histograms obtained from monocyte images. Wherein (A) are the histograms of R channel; (B) are the histograms of (G) channel; and (C) are the histograms of channel B.

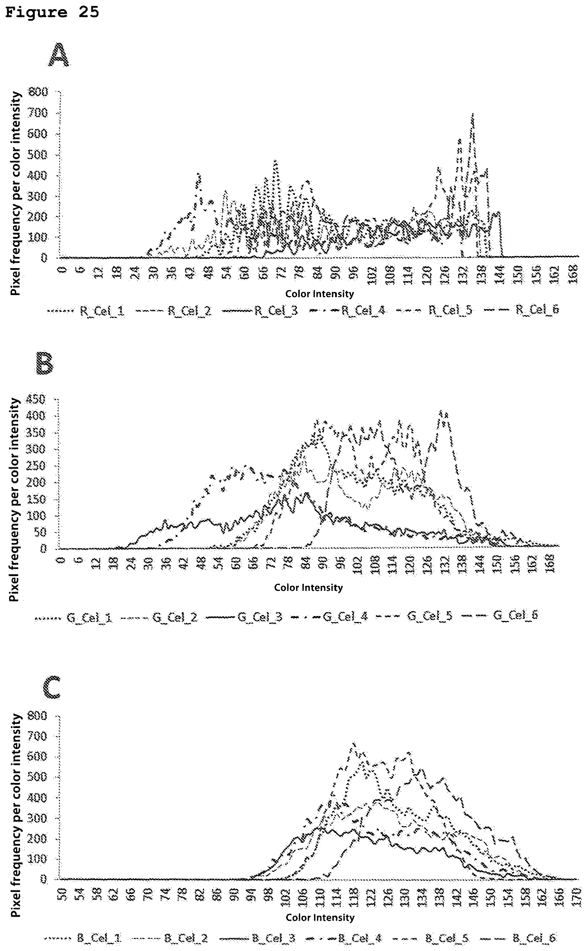

Figure 25 - RGB histograms obtained from eosinophil images. Wherein (A) are the histograms of R channel; (B) are the histograms of (G) channel; and (C) are the histograms of channel B.

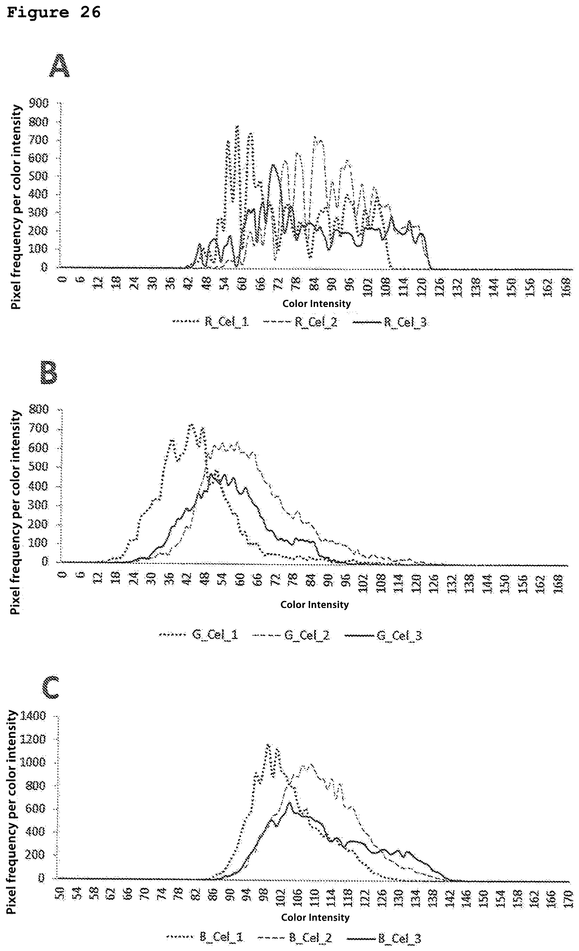

Figure 26 - RGB histogram obtained from basophil image. Wherein (A) are the histograms of R channel; (B) are the histograms of (G) channel; and (C) are the histograms of channel B.

### EXAMPLES

These Examples are merely presented for illustration and should not, in any way, be construed as limiting the range and scope of the present invention.

### Example 1. Preparation of the dye composition

In order to obtain a viable staining in liquid medium, the preparation of the dye solution and the lysing agent is divided into five steps. The purpose of splitting the preparation in stages is achieving a balanced dye solution that meets three requirements for achieving acceptable differential staining for diagnosis: 1) not producing coagulation or particle agglutination; 2) maintaining the integrity of the leukocytes for long enough in order to perform the characterizations and counts; 3) in the case of leukocyte staining from whole blood, allowing hemolysis, without the presence of important cell debris, which may disrupt the recognition and counting of leukocytes. The differential staining protocol in liquid medium is then applied:

### Step 1: Preparation and purification of MG:

In the first step, a buffer solution is prepared in partially isotonic aqueous medium so that it has a buffering power in the pH range of not less than 3.5 and not more than 5.5 in order to obtain the performance of the methyl green dye. The buffer solution should be adjusted for each body fluid type, so that the staining and counting requirements are met. The methyl green is then prepared in the partially isotonic aqueous buffer solution at a concentration of not less than 0.3 mM and not more than 8.0 mM, depending on the type of staining desired and the type of body fluid to be applied. Depending on the origin and formulation of the methyl green, it is necessary to carry out its purification. When purification is needed, the violet crystal contaminant, which is common in powder formulations of methyl green, should be removed. The purification protocol may vary according to the quality of the product that is provided by each manufacturer.

To carry out the purification of methyl green, successive washes of the solution in chloroform are made. Washing is carried out by adding chloroform to the methyl green solution. The violet crystals dissolve in chloroform by stirring. By leaving the solution still, the chloroform with violet crystals are separated in a phase, which is separated in a separating funnel. At the end, a purified solution having a pH of not less than 3.8 and not more than 5.5 should be obtained.

### Step 2: Pironin addition

In the second step, the addition of the dye pironin in the MG solution already purified in concentration of not less than 0.3 mM and not greater than 7.0 mM is carried out. After homogenization, the solution must be filtered to obtain a solution (MGP) with a pH of not less than 3.8 and not higher than 5.5.

### Step 3: Fluorescein preparation

Fluorescein is prepared in the third step, in a hot alcoholic medium, in a concentration of not less than 0.1 mM and not greater than 3.0 mM. After filtration, a solution having a pH of not less than 9 and not more than 10 is obtained.

### Step 4: Preparation of the MGPF solution with addition of fluorescein

In the fourth step the MGPF solution is obtained by mixing the MGP solution with the fluorescein solution in the ratio of not less than one part of MGP to two parts of fluorescein and not more than two and one-half parts of MGP to one part of fluorescein, thus obtaining the MGPF solution with a pH of not less than 4.8 and not more than 6.4. The mixing of the MGP solution with the fluorescein solution should be balanced in order to safely prevent coagulation or the formation of particulates agglutinated in the medium. The balance of the solution should be optimized for the body fluid in which it will be used. This solution is used for staining cells in blood distension or in liquid medium, but without haemolysis. At this step, the dye solution is already viable since it is possible to stain the leukocytes present in most body fluids that do not have too many red blood cells, such as urine, cerebrospinal fluid, cavity liquids, such as ascites, pleural, peritoneal and synovial; and food fluids, such as milk.

### Step 5: Preparation of the final MGPF(H) solution with addition of the lysing agent

Addition of the lysing agent is an alternative for leukocyte staining in whole blood. The lysing agent can be quaternary ammonium compound in the ratio of 5 g/L to 80 g/L associated with a buffer solution and diluted in aqueous medium. This solution is incorporated into the MGPF solution, yielding the MGPF(H) solution. The ratio of MGPF to the lysing agent may be carried out in the range of one part of the lysing agent to two parts of the staining solution up to the ratio of two parts of lysing agent to one part of the staining solution. The ratio should guarantee hemolysis without damage to the leukocytes and allowing their staining. The final dye solution of MGPF(H) should remain at pH not less than 5.5 and not higher than 6.6.

### Example 2: Method of preparing and reading the cells.

As recommended by the usual technique of reading the amounts of leukocytes in liquid medium, a sample of whole blood is diluted in the dye solution in the ratio of one part of blood to 20 parts of the dye solution. By placing this solution in the Neubauer chamber, the stained cells are differently counted. Thus, counting occurs for both the overall value (total leucocytes per cubic millimeter of blood) and for the ratios of each cell type.

The complete protocol can be carried out in less than two minutes by collecting a precise volume of blood by digital puncture, and presents the same results as the counts performed by using an automated counter and through manual methodology, which takes up to 60 minutes until the result of each exam.

The same coloring solution, without the addition of the lysing agent (MGPF) also performs the blood distension staining in one step, but with time of action in the range of 15 to 30 minutes. The time of action depends on the conditions of pH, temperature and fixation technique that were used.

### Example 3. Characterization and evaluation of the stability of dye solutions

The dye solutions which gave rise to the invention, which is the final solution named MGPF(H), were evaluated by spectrophotometry on two dates. The first assessment took place the day after its initial preparation on September 19th, 2014. The same solution was evaluated again, under the same conditions, six months, nine months, twelve months and fifteen months after its preparation. The chemical stability of the solutions, as well as their action on cell staining, remained unchanged until the twelfth month.

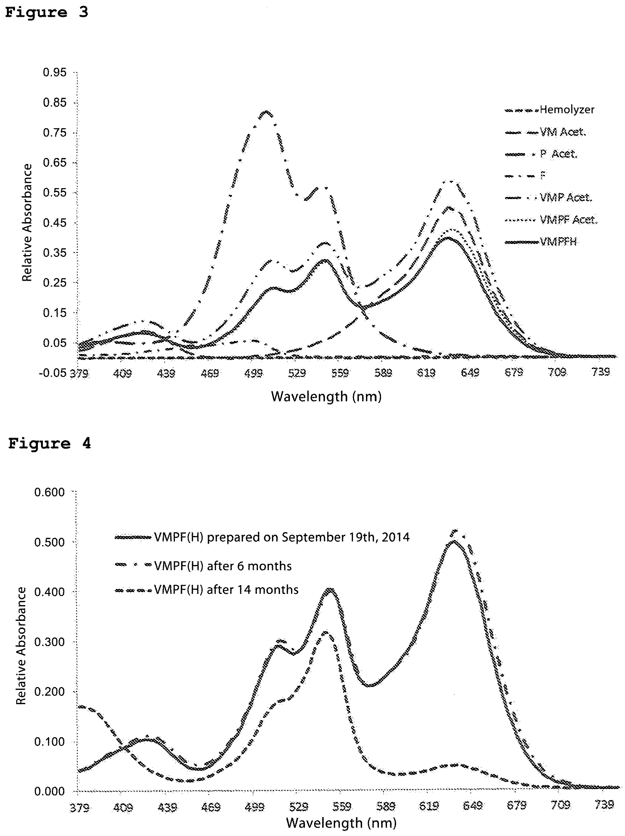

The chemical stability of the dye solutions was verified by absorption spectrophotometry, in two steps. In the first, all individual solutions of each dye were read in the spectrophotometer, in order to evaluate the individual composition of each component. Absorptions of the intermediate dye solutions and the final solution in two MGPF versions (without lysing agent) and MGPF(H) (with lysing agent) were also read. In order to know whether individual solutions react with each other and form other substances. In the second step the chemical stability over time was evaluated, that is, if there is degradation of the final dye solution after long periods of time. In the graph of figure 3, relative values of the absorptions of each solution, and of their combinations, as a function of the wavelength (between 350 nm and 750 nm) are presented. The relative values of the absorptions vary as a function of the dilutions, which were necessary for the spectrophotometer to operate within its working range. Therefore, the absorptions presented are in relative values. Analyzing the absorption curves shown in figure 3, it is shown that the MG in acetate buffer solution has an absorption band between 590 nm and 680 nm, with a peak at 640 nm. Pironin in the buffer solution absorbs in the band between 480 nm and 570 nm, with a peak at 505 nm. Alcoholic fluorescein absorbs in the range of 440 nm to 520 nm, with a peak at 490nm. However, it should be noted that to obtain this spectrum, both MG and pironin were diluted 25 fold, whereas fluorescein did not require dilution. When analyzing the absorbance of the combination of the two MG and P (MGP) cationic dyes, it is observed that there are absorption peaks at 640 nm (corresponding to the wavelength of the MG) and subsidiary peaks at 550 and 505 nm (corresponding to the P length wave) . The same happens when we observe the combination of MG, P and F (MGPF) dyes. However, when the two dyes are associated with fluorescein, this should not be evident. The association of the three dyes was read in the presence of the lysing agent (MGPF (H) line) and without the presence of the lysing agent (MGPF line, which is under the MGPF (H) line). Comparing the MGPF and MGPF(H) lines, it is noted that there are no differences between them, which indicates that the presence of the lysing agent does not interfere with the solution. Thus, it was found that the staining activity of each dye alone was maintained and that each dye reacts with the cell structure or with the molecules with which it has affinity.

In addition, the MGPF (H) line is the overlap of 25x diluted MG lines with 25x diluted pironin (P). And as expected, the fluorescein line (F) (undiluted) cannot be observed in the graph overlap. However, no other peak appears on the absorption line of MGPF (H), indicating that no other substance was formed in the association. The individual absorption spectra and peaks are similar to those obtained by Tycho et al. (1976) and Horobim (2008). The absorbance of the lysing agent is negligible in this range of the spectrum, as observed in figure 3, it does not react with the other components of the solution, so it does not appear in the spectrum of the final solution.

The absorbance spectra of the first step were obtained on September 19th, 2014. For the second step, a reading was performed in the same spectrophotometer, under the same conditions of temperature and relative humidity on March 6th, 2015, of the final solution. The spectra comparing the final solution read on September 19th, 2014, with the same solution read on March 6th, 2015, are shown in figure 4.

### Example 4. Application of the dyeing solution and method of preparation and counting

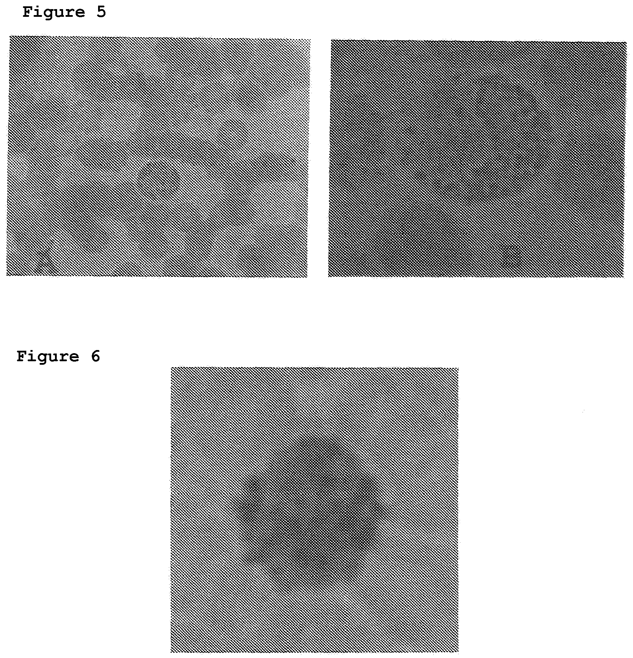

Examples of the staining results are shown in Figures 5 and 6, where it is also possible to observe that a great advantage of this dye solution is the absence of artifacts, fragments or lumps of lysed erythrocytes, which could impair the counting by diverting attention from the professional, or in the case of image processing, simplification in the segmentation step. It is noteworthy that, in liquid medium, the staining did not exceed more than one minute, besides the time inherent to making the slide or the chamber, totaling two minutes.

### Example 5. Evaluation of cells and cell structures in the images obtained in blood distentions stained with MGPF

### Eosinophils

Eosinophils were stained with the MGPF dye solution, as observed in figures 5(a) and 5(b). The slides containing the blood distensions were previously fixed in methanol, which facilitates the entry of fluorescein dye into the cells and the staining of the granules characteristic of this cell type.

As reported by Stankiewicz et al. (1996) the available methods do not allow visualization of the eosinophilic granules in viable cells. In most techniques, eosinophils are stained after being fixed and treated by fixatives that can change the appearance and cell function. Thus, the ability to observe viable eosinophils with their stained granules can provide important information about the activities of these cells. The complete recognition of eosinophils depends on good staining by fluorescein.

### Basophils

When basophils are found in MGP stained distension, as it is shown in figure 6, the granules stain pink due to their histamine content and mainly due to the presence of heparin, a glycosaminoglycan-type heteropolysaccharide (Andréo Filho, 2009), since they have affinity with pironin (TYCHO et al, 1976) .

This morphological and staining behavior also occurs in liquid medium, since the pironin component of the MGPF dye solution showed that it satisfactorily penetrates the cell, for example, demonstrating the RNA content in the lymphocytes stained in liquid medium. Thus, it marks the heparin content of basophils by their affinity.

### Example 6. Evaluation of cells and cell structures in the images obtained in liquid medium

To exemplify the results obtained with this invention, peripheral blood leukocytes were identified, classified and counted in five morphological types: neutrophils, lymphocytes, monocytes, eosinophils and basophils.

To exemplify the results obtained with this invention, leukocytes present in other body fluids: urine, liquor (cerebrospinal fluid), cavity liquids, such as ascites, pleural, peritoneal and synovial, were also characterized and categorized by means of their images. For purposes of characterization, classification and counting, the images of the cells contained in these liquids present the same characteristics and information as the images of the cells contained in the blood. In order to obtain adequate staining in these liquids, it was necessary to adjust the pH and/or concentration of the final solution as well as centrifugation of the liquids for concentration of the cell volume. The staining occurs in the presence of the two solutions: MGPF and MGPF(H).

The observation and description of the cells below corresponds to that found in the liquid readings between slide and coverslip, stained with MGPF(H) or MGPF using a 1000X magnification.

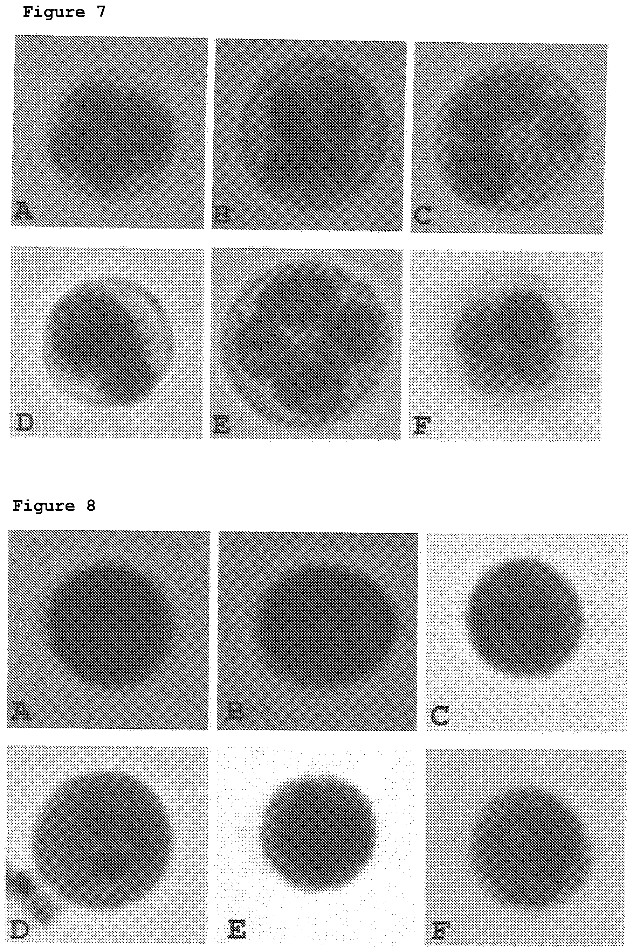

Cells were visualized in their characteristics spherical shape and size, showing no rupture of cell membranes. The images presented in figures 7 to 11 had their relative sizes preserved, i.e., considering that all images had the same magnification (1000X), the areas cropped around the cell were always the same (500x500 pixels). Therefore, the cell area is proportional to the size of the cutting window.

### Neutrophils

In this cell, the cytoplasm was light, clear and without granulation. The nucleus appeared in its characteristic segmentation form. Both rods and polymorphonuclear cells were classified as neutrophils. Nuclei with 2 to 4 segmentations could be observed, as it can be observed in the examples presented in figure 7. Due to the affinity of the MG component with DNA, the nuclear segments became bluish-green.

### Lymphocytes

It can be observed in this cell the characteristic of high nucleus cytoplasm ratio, with evident nuclear observation stained in intense blue, as it is seen in figure 8. During the counts, it was possible to observe sharp nucleoli in some cells, as seen in figure 8(d). The nucleoli presented a reddish coloration that outlined the nucleus, which can be related to the affinity of the pironin for RNA, present in such structure. In slides of Giemsa stained blood distensions, this finding is not common (BEUTLER, 2001). Cytoplasmic bands stained with different pink intensities confirmed the affinity of the pironin component of the MGPF(H) or MGPF solution for RNA, characterizing differences in cell activity. No cytoplasmic granules were observed.

### Monocytes

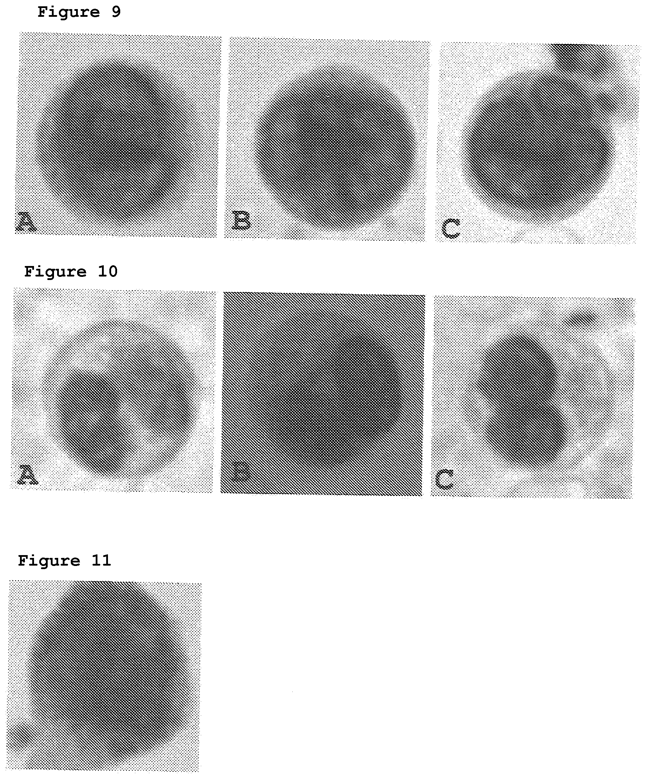

These cells were shown with a wide variety of nuclear and cytoplasmic forms, as it is shown in figure 9. Generally, they appeared as large cells, confirming their description as read in RAWAT et al. (2015) . The nuclei were visualized in several forms, ranging from rounded, reniform, oval, lobed and even folded. Nucleoli were not evidenced. The cytoplasm sometimes appeared vacuolated and with a fine granulation stained in loose reddish-pink, by the affinity with pironin, since it represents its RNA content. This staining tends to be less intense than in reactive lymphocytes, which may aid in the visual differentiation between monocytes and these lymphocytes, corroborating the descriptions presented by Beutler (Beutler, 2001).

### Eosinophils

The identification of these cells took into account some of their normally reported characteristics, such as cell size, considered to be medium between granulocytes: neutrophils and basophils, the low nucleus cytoplasm ratio, the nucleus location, usually eccentric, with bilobular segmentation most often and absence of visible nucleoli, as observed in figure 10. The yellowish cytoplasmic staining, produced by fluorescein, and the bluish-green staining produced by MG, weaker than the nucleus, differentiates these cells from the neutrophils.

### Basophils

The description of the morphological and staining characteristics of this cell type, stained with MGPF(H) or MGPF in liquid medium, are very similar to those found in the classic literature, as shown in figure 11. Due to the large amount of heparin rich cytoplasmic granules, which the basophil has and the affinity of the pironin component for the glycosaminoglycan, the cells referenced are richly stained in intense pink, almost disappearing with the nucleus, which is slightly bluish under the substantially colored cytoplasm. In a more detailed testing, it can be seen that the cytoplasm staining refers to the large granules, which are not as dark as in the classical staining of Romanóvisk. The diversity of characteristics in relation to other cells and their scarcity, less than 1% of total leukocytes, contributes to this classification.

### Example 7: Evaluation of dye solution by comparing counts of cells with established protocols and with the new dye.

In order to evaluate the applicability of the MGPF(H) dye solution as a differential leukocyte detection instrument, it is necessary to determine if the counts performed are compatible with the counts performed by the established methodologies. Then, global counting and differential counting were evaluated by ANOVA variance analysis and by Blend-Altman agreement analysis.

### Global Count

For each blood sample, three global counts were performed, one by the Laboratory of Clinical Analysis (LAC), the second made by the inventors in a Neubauer chamber with Turk's solution and the third made by the inventors using MGPF(H).

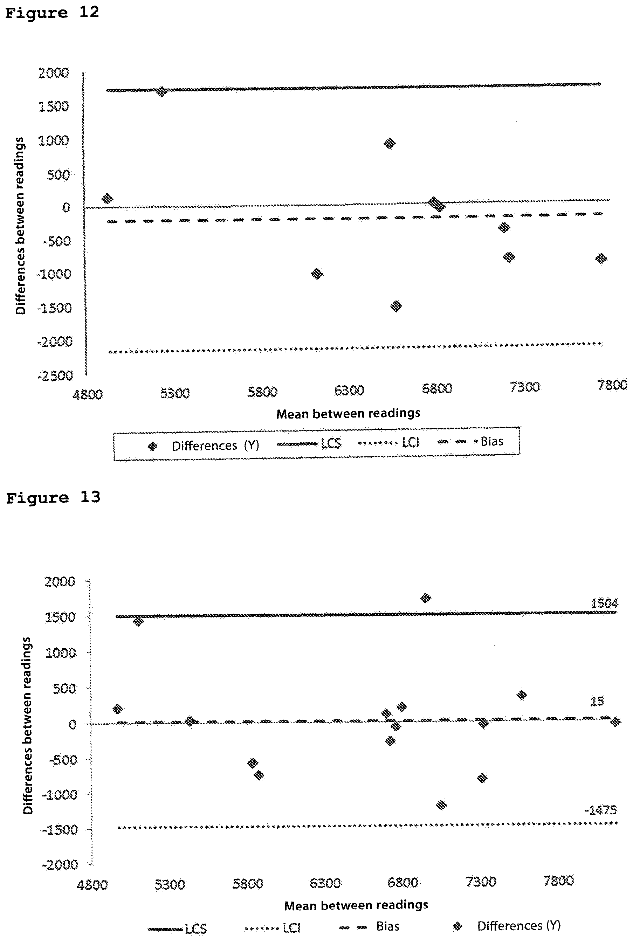

The results of these counts are presented in table 1. Next to the table the results obtained by ANOVA analysis are presented. Figures 12 and 13 show the Blend-Altman analyzes for MGPF(H) x LCA and MGPF(H) x Turk's solution. No significant statistical differences were found between counting techniques, through ANOVA and Blend-Altman, thus disclosing that they are equivalent.

**Table 1 - Result of the global leukocyte count by three techniques: Automatic, performed by an independent Clinical Analysis Laboratory. Manual in Neubauer chamber with Turk's solution. Manual in Neubauer chamber with MGPF(H). Values expressed in cells/mm³.**

| **Sample** | **LCA** | **turk** | **MGPF(H)** |
|---|---|---|---|
| Patient 1 | 7000 | 7750 | 7400 |
| Patient 2 | 5000 | 5075 | 4875 |
| Patient 3 | 7300 | 8150 | 8200 |
| Patient 4 | 7000 | 7825 | 6100 |
| Patient 5 | 6800 | 6725 | 6800 |
| Patient 6 | 6800 | 6588 | 6867 |
| Patient 7 | 5600 | 6750 | 6650 |
| Patient 8 | 5800 | 7300 | 7350 |
| Patient 9 | 6100 | 5825 | 4400 |
| Patient 10 | 6800 | 6450 | 7650 |
| Patient 11 | 5600 | 5450 | 5425 |
| Patient 12 | 5200 | 5500 | 6250 |
| Patient 13 | 5500 | 5550 | 6125 |
| Patient 14 | 7000 | 6900 | 7725 |
| Patient 15 | 6700 | 6900 | 6700 |

The results of the one-way ANOVA test were: p = 0.62, with critical F greater than 3.35, and F was 0.50. According to the test, if the p value is greater than 0.05 and F is smaller than the critical F, the counts are not statistically different, that is, they can be considered equivalent.

### Differential Counting

For each blood sample, differential counts of leukocytes were performed by three techniques, one automated by LCA, the second by the inventors, manually using MGPF (H) and the third by the inventors, carried out manually, in Leishman-stained blood distension.

### Neutrophils

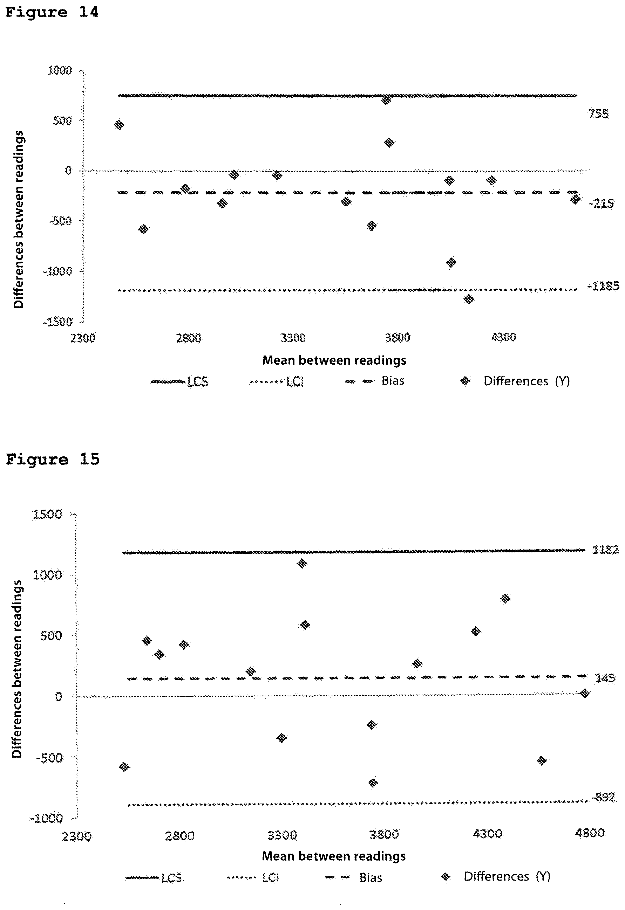

The results of these counts are presented in table 2. Next to the table the results obtained by ANOVA test are presented. Figures 14 and 15 show the Blend-Altman analyzes for LCA x MGPF(H) and Leishman x MGPF(H) . No significant statistical differences were found between counting techniques, through ANOVA and Blend-Altman, thus disclosing that they are equivalent.

**Table 2 - Result of the neutrophil differential counting by three techniques: Automatic, performed by an independent Clinical Analysis Laboratory. Manual on Leishman-stained blood distension. Manual with MGPF(H). Values expressed in total number of cells/mm3.**

| **Sample** | **LCA** | **MGPF(H)** | **Leish.** |
|---|---|---|---|
| Patient 1 | 4200 | 4292 | 4844 |
| Patient 2 | 2700 | 2876 | 2538 |
| Patient 3 | 3600 | 4510 | 3994 |
| Patient 4 | 4100 | 3386 | 4108 |
| Patient 5 | 3400 | 3706 | 3127 |
| Patient 6 | 3400 | 3948 | 2866 |
| Patient 7 | 2800 | 3126 | 3476 |
| Patient 8 | 3500 | 4778 | 4782 |
| Patient 9 | 2700 | 2244 | 2825 |
| Patient 10 | 4000 | 4093 | 3838 |
| Patient 11 | 3000 | 3038 | 2616 |
| Patient 12 | 2300 | 2875 | 2420 |
| Patient 13 | 3200 | 3246 | 3053 |
| Patient 14 | 4500 | 4790 | 4002 |
| Patient 15 | 3900 | 3618 | 3864 |

The results of the one-way ANOVA test were: p = 0.77, with critical F greater than 2.6, and F was 0.27. The p value much greater than 0.05 and F lower than the critical F indicates that the counts do not present statistical differences, that is, they can be considered equivalent.

### Lymphocytes

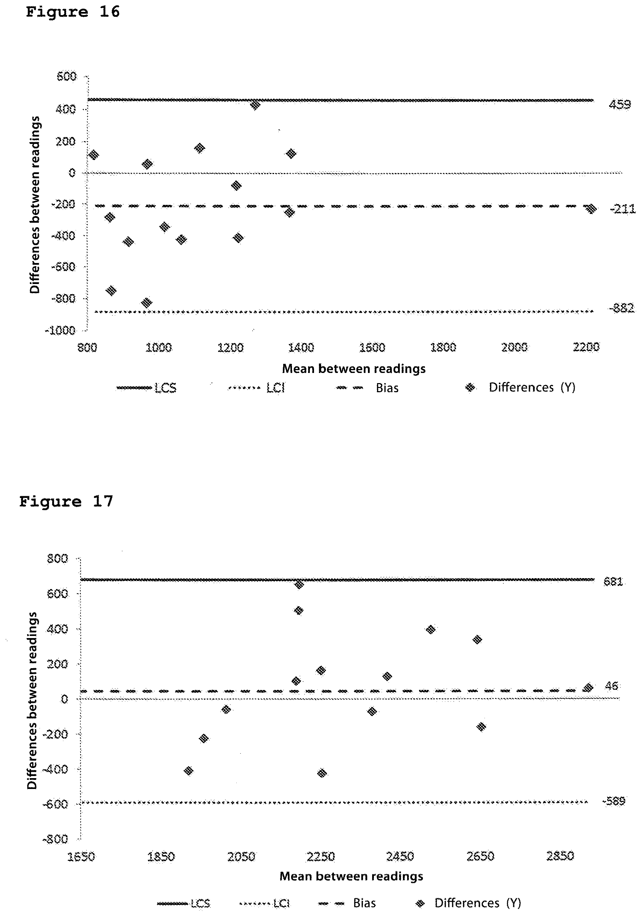

The results of these counts are presented in table 3. Next to the table the results obtained by ANOVA test are presented. Figures 16 and 17 show the Blend-Altman analyzes for LCA x MGPF(H) and Leishman x MGPF(H). No significant statistical differences were found between counting techniques, through ANOVA and Blend-Altman, thus disclosing that they are equivalent.

**Table 3 - Result of the lymphocyte differential counting by three techniques: Automatic, performed by an independent Clinical Analysis Laboratory. Manual on Leishman-stained blood distension. Manual with MGPF(H). Values expressed in total number of cells/mm3.**

| **Sample** | **LCA** | **MGPF(H)** | **Leish.** |
|---|---|---|---|
| patient1 | 2100 | 2331 | 2170 |
| patient2 | 1600 | 1487 | 1802 |
| patient3 | 2700 | 2952 | 2893 |
| patient4 | 2200 | 2044 | 2465 |
| patient5 | 2400 | 2482 | 2354 |
| patient6 | 2700 | 2575 | 2734 |
| patient7 | 1900 | 2727 | 2329 |
| patient8 | 1700 | 1985 | 2044 |
| patient9 | 2500 | 1716 | 2126 |
| patient10 | 2100 | 2525 | 1871 |
| patient11 | 1900 | 1845 | 2071 |
| patient12 | 2400 | 2813 | 2475 |
| patient13 | 1700 | 2450 | 1943 |
| patient14 | 1800 | 2240 | 2139 |
| patient15 | 2000 | 2345 | 2415 |

The results of the one-way ANOVA test were: p = 0.51, with critical F greater than 2.6, and F was 0.73. The p value much greater than 0.05 and F lower than the critical F indicates that the counts do not present statistical differences, that is, they can be considered equivalent.

### Monocytes

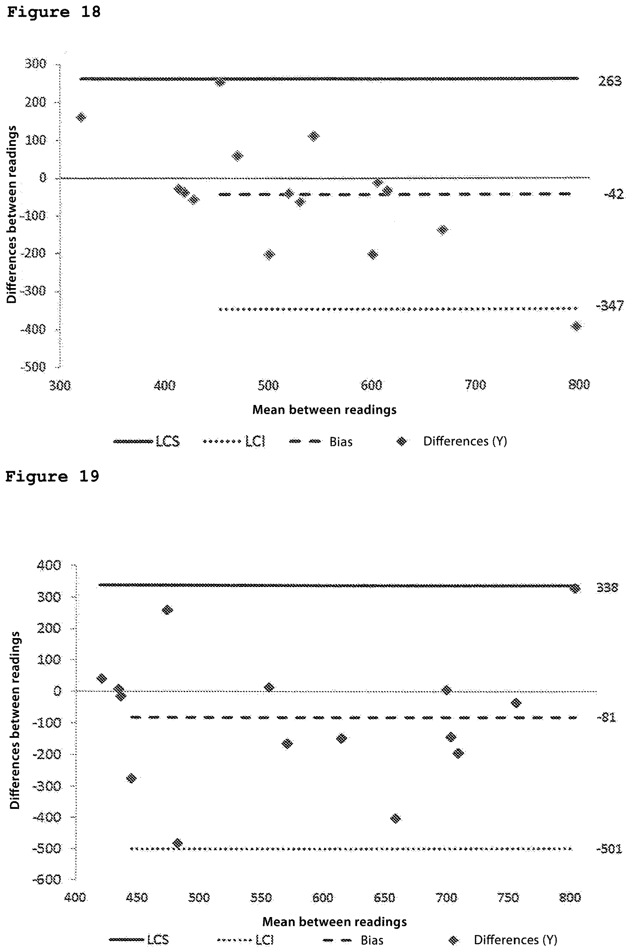

The results of these counts are presented in table 4. Next to the table the results obtained by ANOVA test are presented. Figures 18 and 19 show the Blend-Altman analyzes for LCA x MGPF(H) and Leishman x MGPF(H). No significant statistical differences were found between counting techniques, through ANOVA and Blend-Altman, thus disclosing that they are equivalent.

**Table 4 - Result of the monocyte differential counting by three techniques: Automatic, performed by an independent Clinical Analysis Laboratory. Leishman-stained blood distension. Manual stained with MGPF(H). Values expressed in total number of cells/mm3.**

| **Sample** | **LCA** | **MGPF(H)** | **Leish.** |
|---|---|---|---|
| Patient 1 | 500 | 703 | 698 |
| Patient 2 | 400 | 439 | 431 |
| Patient 3 | 600 | 738 | 774 |
| Patient 4 | 400 | 458 | 861 |
| Patient 5 | 600 | 612 | 807 |
| Patient 6 | 400 | 240 | 725 |
| Patient 7 | 600 | 632 | 776 |
| Patient 8 | 500 | 441 | 402 |
| Patient 9 | 600 | 308 | 583 |
| Patient 10 | 600 | 995 | 613 |
| Patient 11 | 600 | 488 | 654 |
| Patient 12 | 500 | 563 | 550 |
| Patient 13 | 400 | 429 | 444 |
| Patient 14 | 500 | 541 | 690 |
| Patient 15 | 400 | 603 | 345 |

The ANOVA test results were: p = 0.10, with critical F greater than 2.45, and the obtained F was 2.3. The p value much greater than 0.05 and F lower than the critical F indicates that the counts do not present statistical differences, that is, they can be considered equivalent.

According to Koepke (1977) apud England (1979), monocyte counts show a high variability, due in large part to an expected variation, but mainly due to differences in counting techniques, since normal or reactive lymphocytes may be erroneously identified as monocytes, making the monocyte count sometimes unsatisfactory. Another source of interference in the statistical analysis are the results presented by the LCA, which occur in multiples of 100. Assuming that a reading error of about 50 cells may occur and given that this error represents a significant percentage of the total cells, the statistical results tend to diverge from the expected values.

### Eosinophils

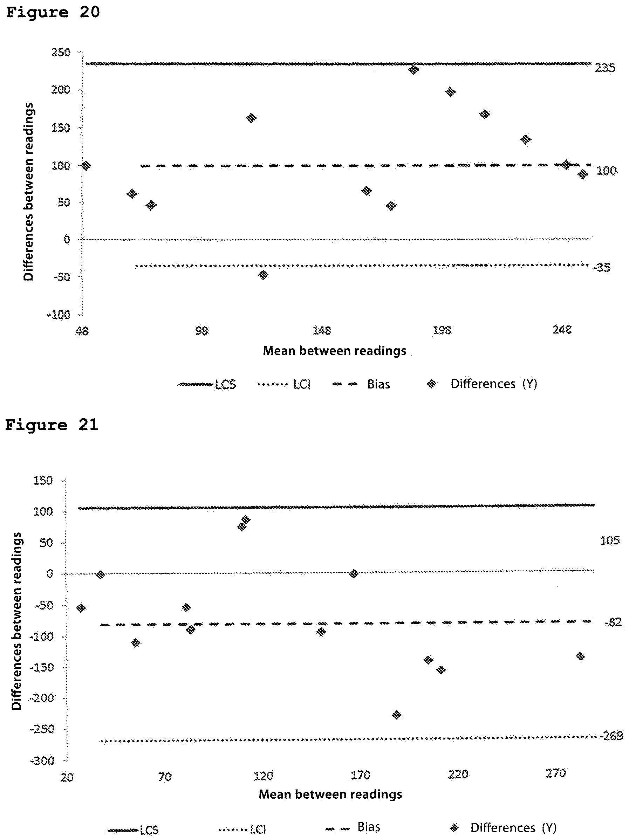

The results of these counts are presented in table 5. Figures 20 and 21 show the Bland-Altman analyzes for LCA x MGPF(H) and Leishman x MGPF (H) . However, due to the low number of cells available per patient and the fact that the LCA maintains the count in multiples of 100, it is not possible to perform an appropriate statistical analysis. Then, the counts data are presented based on the characterization and visual recognition of the cells by experienced hematologists.

**Table 5 - Result of the eosinophil differential counting by three techniques: Automatic, performed by an independent Clinical Analysis Laboratory. Leishman-stained blood distension. Manual with MGPF(H). Values expressed in total number of cells/mm3.**

| **Sample** | **LCA** | **MGPF(H)** | **Leish.** |
|---|---|---|---|
| Patient 1 | 200 | 37 | 38,8 |
| Patient 2 | 300 | 73 | 304 |
| Patient 3 | 300 | 200 | 448 |
| Patient 4 | 300 | 213 | 352 |
| Patient 5 | 400 | 350 | 403 |
| Patient 6 | 300 | 103 | 197 |
| Patient 7 | 300 | 166 | 168 |
| Patient 8 | 100 | 147 | 73 |
| Patient 9 | 300 | 132 | 291 |
| Patient 10 | 100 | 38 | 129 |
| Patient 11 | 100 | 54 | 109 |
| Patient 12 | 100 | 22 | 55 |
| Patient 13 | 100 | 64 | 111 |
| Patient 14 | 200 | 154 | 69 |
| Patient 15 | 200 | 134 | 276 |

### Basophils

Basophils are rare cells, and their count usually results in null values in most blood counts. As LCA maintains the count in multiples of 100, the result for most patients was null for absolute values, and non-zero values were presented in the relative results (as a percentage of total leukocytes), as shown in table 6. Thus, Bland-Altman's statistical analysis or concordance analysis is meaningless. Then, the counts data are presented based on the characterization and visual recognition of the cells by experienced hematologists.

**Table 6 - Result of the monocyte differential counting by three techniques: Automatic, performed by an independent Clinical Analysis Laboratory. Leishman-stained blood distension. Manual with MGPF(H). Values expressed as a percentage of total leukocytes.**

| **Sample** | **LCA** | **MGPF(H)** | **Leish.** |
|---|---|---|---|
| Patient 1 | 0.4 | 0.5 | 0.0 |
| Patient 2 | 0.6 | 0.2 | 0.0 |
| Patient 3 | 0.5 | 0.3 | 0.5 |
| Patient 4 | 0.3 | 0.1 | 0.5 |
| Patient 5 | 0.2 | 0.1 | 0.5 |
| Patient 6 | 0.2 | 0.0 | 1.0 |
| Patient 7 | 0.3 | 0.2 | 0.3 |
| Patient 8 | 0.4 | 0.3 | 0.2 |
| Patient 9 | 0.4 | 0.2 | 0.3 |
| Patient 10 | 0.4 | 0.2 | 0.0 |
| Patient 11 | 0.5 | 0.3 | 0.3 |
| Patient 12 | 0.3 | 0.2 | 0.2 |
| Patient 13 | 0.3 | 0.3 | 0.3 |
| Patient 14 | 0.2 | 0.1 | 0.2 |
| Patient 15 | 0.6 | 0.5 | 0.5 |

### Example 8: Evaluation of the effectiveness of the method of cell classification and counting by image processing.

In order to evaluate the effectiveness of this automatic leukocyte classification method, 226 cell images were processed, containing samples of the five leukocyte cell types and 20 non-cell artifacts.

The histogram metrics, such as: width of the bases of intensities; relative position of frequency peaks; order of occurrence of the first and last valid frequencies in Red (Red), Green (Green) and Blue (Blue) (RGB), that is, greater than 0 (Zero); peak shift to the right or left, allow the characterization of the cell types.

The same images were evaluated by experienced biomedicians in leukocyte classification. The comparative results of classification and automatic counting with biomedical classification and counting are presented in table 7, whose R2 correlation between biomedical and software classification is equal to 0.998, showing that the counts are equivalent.

### Table 7 - Comparison of leukocyte counts performed by biomedical and image processing software.

Linear correlation (R²) equal to 0.998.

| | **Biomedical Classification** | **Software Classification** |
|---|---|---|
| Neutrophil | 97 | 96 |
| Lymphocyte | 65 | 63 |
| Monocyte | 35 | 38 |
| Eosinophils | 8 | 7 |
| Basophils | 2 | 2 |
| NC | 19 | 20 |
| **Total** | **226** | **226** |

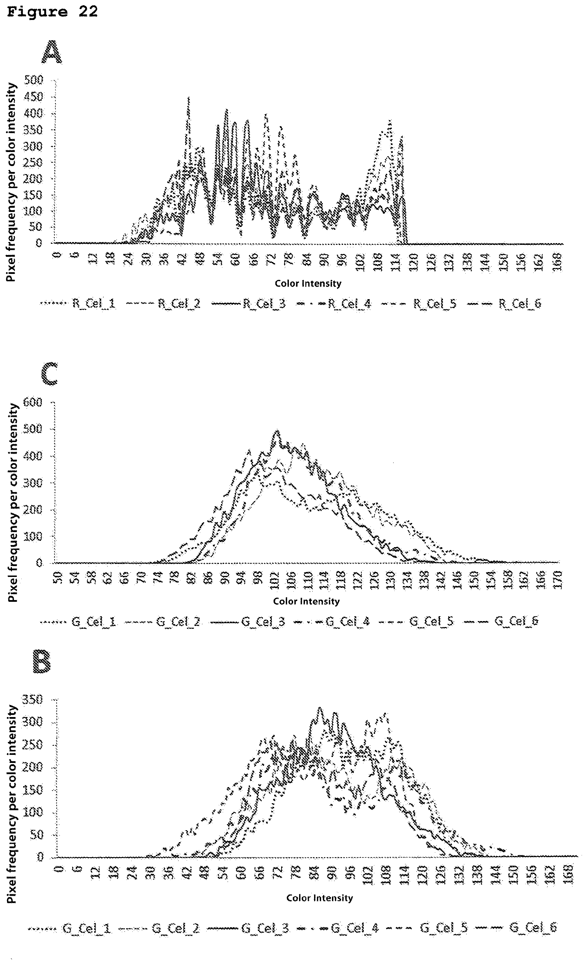

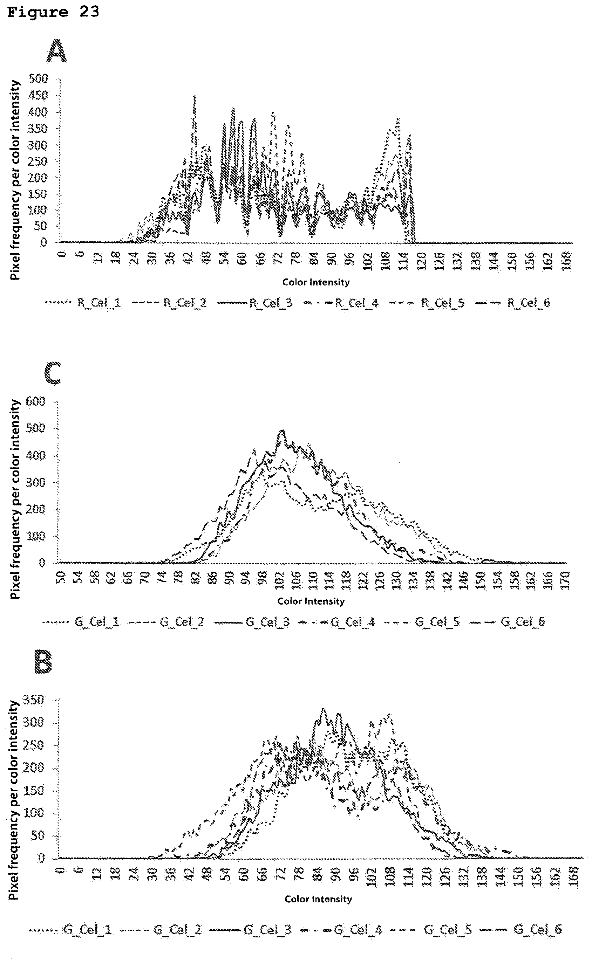

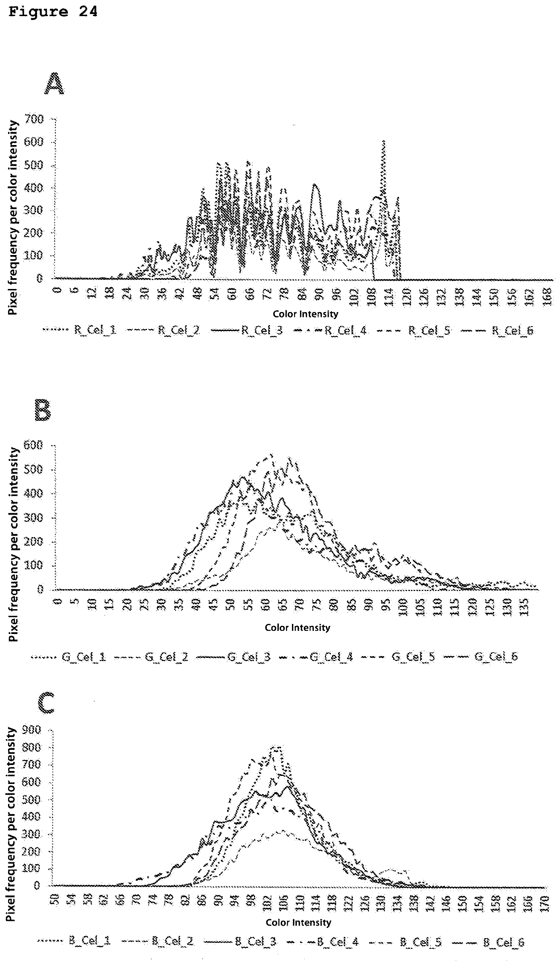

Figure 20 shows the uniformity of neutrophil histogram behavior, which is different from the uniformity of the histograms obtained from lymphocyte images (Figure 21), from monocyte images (Figure 22), from eosinophil images (Figure 23) and from basophil images (Figure 24).

### References

ADOLLAH, R.; MASHOR, M. Y.; NASIR, N. F. M.; ROSLINE, H.; MAHSIN, H.; ADILAH, H. Blood cell image segmentation: a review. In: 4th Kuala Lumpur International Conference on Biomedical Engineering 2008. Springer Berlin Heidelberg, 2008. pages 141-144.
ANDREO FILHO, Newton. Challenges in the quality of heparin. Revista Brasileira de Hematologia e Hemoterapia, v. 31, n. 5, pages 306-307, 2009.
BAIN B. J. Células Sanguíneas: Um Guia Pratico. 4th ed. Porto Alegre: Artmed, 2007. 487 p.
BANCROFT, J. D.; SUVARNA, S. K.; LAYTON, C. Bancroft's Theory and Practice of Histological Techniques. 7th ed. Elsevier, 2013. 654 p.
BASTEN, Antony; BOYER, Markley H.; BEESON, Paul B. Mechanism of eosinophilia I. Factors affecting the eosinophil response of rats to Trichinella spiralis. The Journal of experimental medicine, v. 131, n. 6, pages 1271-1287, 1970.
BEUTLER Ernest , LICHTMAN Marshall A. , COLLER Barry S. , KIPPS Thomas J. , SELIGSOHN Uri .Willians Hematology. 6th ed. McGraw-Hill, Medical Publishing Division. 2001. 2047 p
CARRERAS, Maria Cecilia et al. Kinetics of nitric oxide and hydrogen peroxide production and formation of peroxynitrite during the respiratory burst of human neutrophils. FEBS letters, v. 341, n. 1, p. 65-68, 1994.
DE LA RUE, Mario Luiz. Eosinofilia devida a parasitas. RBAC, v. 33, n. 4, p. 221-223, 2001.
DUCREST, S. et al. Flowcytometric analysis of basophil counts in human blood and inaccuracy of hematology analyzers. Allergy, v. 60, n. 11, p. 1446-1450, 2005.
ENGLAND, J. M. Prospects for automated differential leucocyte counting in the routine laboratory. Clinical & Laboratory Haematology, v. 1, n. 4, p. 263-273, 1979.
FALCONE, Franco H.; HAAS, Helmut; GIBBS, Bernhard F. The human basophil: a new appreciation of its role in immune responses. Blood, v. 96, n. 13, p. 4028-4038, 2000.
GROTTO, Helena ZW. O hemograma: importância para a interpretação da biópsia. Rev. bras. hematol. hemoter, v. 31, n. 3, p. 178-182, 2009.
HOROBIN, R.W.; KIERNAN, J.A. CONN'S Biological Stains. 10. ed. New York: Taylor&Francis, 2008. 555 p.
KIERNAN, John Alan. Histological and histochemical methods: theory and practice. Shock, v. 12, n. 6, pages 479, 1999.
KOEPKE, J. A. A delineation of performance criteria for the differentiation of leukocytes. American journal of clinical pathology, v. 68, n. 1 Suppl, p. 202-206, 1977. apud ENGLAND, J. M. Prospects for automated differential leucocyte counting in the routine laboratory. Clinical & Laboratory Haematology, v. 1, n. 4, p. 263-273, 1979.
KURIMOTO, Yoshiyuki; DE WECK, A. L.; DAHINDEN, C. A. Interleukin 3-dependent mediator release in basophils triggered by C5a. The Journal of experimental medicine, v. 170, n. 2, p. 467-479, 1989.
LEISHMAN, W. B. Note on a simple and rapid method of producing Romanowsky staining in malarial and other blood films. British medical journal, v. 2, n. 2125, p. 757, 1901.
LIMA, Adriana; et al. Efeito do Solvente sobre as Propriedades Espectroscópicas do Azul de Metileno. In: XI Encontro Latino Americano de Iniciação Científica e VII Encontro Latino Americano de Pós-Graduação - Universidade do Vale do Paraíba. 2007
MAHAJAN, S.; GOLATI, S. S.; MESHRAM, A.; JICHLKAN, N. Review: Detection of Types of Acute Leukemia. International Journal of Computer Science and Mobile Computing, v.3, n. 3, p. 104-111, March 2014.
MARIEB, E. N. Essentials of human anatomy and physiology, ed 10, New York: Pearson, 2011.
MARSHALL, P. N.; BENTLEY, S. A.; LEWIS, S. M. A standardized Romanowsky stain prepared from purified dyes. Journal of clinical pathology, v. 28, n. 11, p. 920-923, 1975.
McEwen, B. J. Eosinophils: A review. Vet. Res. Communic. 16: 11-42, 1992. apud Mario Luiz de la Rue; RBAC, vol. 33(4): 221-223, 2001)
MIRČIĆ, Stanislav; JORGOVANOVIC, Nikola. Automatic classification of leukocytes. Journal of automatic control, v. 16, n. 1, p. 29-32, 2006.
NASCIMENTO, V. A. O. ; MORAES T. F.; OLIVEIRA H. J. Q.; Coloração de leucócitos em meio líquido para aquisição e diferenciação por processamento de imagens. In: XXIV Congresso Brasileiro de Engenharia Biomédica - CBEB Uberlândia - MG. 2014
OLIVEIRA, Raimundo Antonio Gomes. Hemograma: como fazer e interpretar. São Paulo: LPM, 2007. 505 p.
ORNSTEIN, LEONARD; ANSLEY, HUDSON R. Spectral matching of classical cytochemistry to automated cytology. Journal of Histochemistry & Cytochemistry, v. 22, n. 7, p. 453-469, 1974.
OVALLE, W.; NAHIRNEY, P. C. Netter Bases da Histologia . 2nd ed. Elsevier, 2014. 536 p
PATERNITI, Irene et al. PDE 7 inhibitors: new potential drugs for the therapy of spinal cord injury. PLoS One, v. 6, n. 1, p. e15937, 2011.
PERRY, SEYMOUR; REYNOLDS, JOHN; BAKER, MARY. Methyl-green-pyronin as a differential nucleic acid stain for peripheral blood smears. Blood, v. 11, n. 12, p. 1132-1139, 1956.
PIURI, Vincenzo; SCOTTI, Fabio. Morphological Classification of Blood Leucocytes by Microscope Image. In: CIMSA 2004-IEEE International Conference on Computational Intelligence for Measurement Systems and Applications. 2004.
POGGERE, Paula Andreia et al. Azul de Metileno: Propriedades e Tratamentos. **Anais do III ENDICT-Encontro de Divulgação Científica e Tecnológica. Universidade Tecnológica Federal do Paraná-Câmpus Toledo,** 2011. www.utfpr.edu.br/toledo
PYÖRÄLÄ, S.; Indicators of Inflammation in the Diagnosis of Mastitis. Veterinary Research, v.34, p. 565-578, 2003.
RAWAT, J.; BHADAURIA, H.S.; SINGH, A.; VIRMANI, J. Review of Leukocyte Classification Techniques for Microscopic Blood Images. In: , 2015 2nd International Conference on Computing for Sustainable Global Development (INDIA Com) p 1948-1954, 2015
ROSENFELD, Ricardo. Complete blood count. J. bras. patol. med. lab, v. 48, n. 4, p. 244-244, 2012.
ROWLEY, Anne H. et al. RNA-containing cytoplasmic inclusion bodies in ciliated bronchial epithelium months to years after acute Kawasaki disease. PLoS ONE 3(2):. 2008.
STANKIEWICZ, M. et al. Supravital staining of eosinophils. International journal for parasitology, v. 26, n. 4, p. 445-446, 1996.
SPRY, C.J.F. Synthesis and secretion of eosinophil granule substances. Immunology today, v. 6, n. 11, p. 332-335, 1985. apud De La Rue; RBAC, vol. 33(4): 221-223, 2001.
SPRY, C. J. F. Eosinophyl leucocytes p.329-332. In: MCGEE, James O.'D.; ISAACSON, Peter G.; WRIGHT, Nicholas A. (Ed.).Oxford Textbook of Pathology: v. 2a,b. Pathology of systems. Oxford University Press, 1992.
TYCKO, D. H. et al. Automatic leukocyte classification using cytochemically stained smears. Journal of Histochemistry & Cytochemistry, v. 24, n. 1, p. 178-194, 1976.
WITTEKIND, D. On the nature of Romanowsky dyes and the Romanowsky-Giemsa effect. Clinical & Laboratory Haematology, v. 1, n. 4, p. 247-262, 1979.

## Claims

1. Cell dye composition **characterized in that** it comprises methyl green or an equivalent of its chemical group triarylmethanes (or triphenylmethanes), pironin or an equivalent of its chemical group xanthenes, and fluorescein or an equivalent of its chemical group fluoroma, or salts and complexes thereof, in a predominantly aqueous medium.

2. Composition, according to claim 1, **characterized in that** it further comprises a cell lysing agent.

3. Composition, according to claim 1 or 2, **characterized in that** it comprises:
- 0.3 mM to 8 mM methyl green;
- 0.3 mM to 8 mM pironin;
- 0.1 mM to 3 mM fluorescein; wherein methyl green, pironin and fluorescein and, optionally, a lysing agent, form a complex in predominantly aqueous medium.

4. Composition, according to any one of claims 1 to 3, **characterized in that** it comprises alcohol concentration of less than or equal to 35%.

5. Composition, according to any one of claims 1 to 4, **characterized in that** the cell lysing agent is selected from the group consisting of quaternary agents capable of disrupting the membranes of the red blood cells, previously diluted in aqueous medium, at the concentrations from 25 g/L to 80 g/L and from 2 g/L to 9 g/L, respectively.

6. Process for the preparation of a dye composition and a lysing agent, as defined in any one of claims 1 to 5, **characterized in that** it comprises the steps of:
a) preparing a MGP solution comprising methyl green and pironin in a concentration from 0.3 mM to 8 mM and from 0.3 mM to 8 mM, respectively, and a pH of 3.8 to 5.5;
b) adding fluorescein to the MGP solution in a ratio of one part of MGP to two parts of fluorescein in the concentration of 0.1 mM to 3 mM, and two and a half parts of MGP to one part of fluorescein, thus obtaining the MGPF solution with pH between 4.8 and 6.4; and
c) optionally, adding a lysing agent in a ratio of one part of the lysing agent to two parts of the MGPF solution to two parts of lysing agent to one part of MGPF solution, wherein the final dyeing solution of MGPF(H) should remain at pH between 5.5 and 6.6.

7. Method of preparing a sample of cells **characterized in that** it comprises cell lysing and differential staining of cell types in a single stage in aqueous medium.

8. Method, according to claim 7, **characterized in that** the single step comprises mixing a sample of cells with a dye composition, as defined in any one of claims 1 to 5.

9. Method, according to claim 8, **characterized in that**, when mixing a sample of cells with a dye composition, as defined in any one of claims 1 to 5, the permeabilization of the cell membrane by the dye composition occurs in aqueous medium, enabling the dye penetration and staining inside the cells.

10. Method, according to claim 7, **characterized in that** mixing a sample of cells with a dye and lysis composition is done at a ratio of one part of body fluid to 5 to 200 parts of the dye composition, depending on the type of body fluid to be analyzed.

11. Method of counting cells, **characterized in that** it comprises the steps of:
a) mixing a sample of cells with a dye and lysis composition, as defined in any one of claims 1 to 4;
b) placing the sample mixture of cells with the dye and lysis composition and a cell counting apparatus;
c) performing the counting of the total value of the cell units, identifying determination of the proportions of each cell subtype in the body fluid sample in a single step.

12. Cell counting method, **characterized in that** it comprises (i) imaging the cells suspended in the dye composition; (ii) global cell type counting and differential counting of cell subtypes in a single step.

13. Method, according to claim 12, **characterized in that** the counting is done automatically by image processing, or non-automatically, by an individual using a microscope.

14. Method, according to claim 11, **characterized in that** it further comprises the step of registering images of the stained and lysed cells and storing such images for future evaluation.

15. Use of a composition as defined in any one of claims 1 to 5 **characterized in that** it is for the preparation of a sample of cells for cell analysis.
